# EUROPEAN PATENT APPLICATION

(11) **EP 4 226 896 A1**
(43) Date of publication of application: **16.08.2023**
(21) Application number: 21876959.4
(22) Date of filing: 28.09.2021
(51) Int. Cl.: A61F 2/24, A61F 2/958, A61F 2/962, A61F 2/95, A61F 2/07

(54) **CONVEYING SYSTEM FOR IMPLANTABLE MEDICAL APPARATUS AND CONTROL HANDLE THEREOF, AND IMPLANTABLE MEDICAL APPARATUS AND FIXING METHOD, LOADING METHOD AND RELEASING METHOD THEREFOR**

(30) Priority: 06.10.2020 US 202017064032
(71) Applicant: Venus MedTech (HangZhou), Inc., Hangzhou, Zhejiang 310052 (CN)
(72) Inventor: PHAM, Trinh Van, Westminster, California 92683 (US); ZENG, Ashley Rachel, Irvine, California 92603 (US); WANG, Xiang, Hangzhou, Zhejiang 310052 (CN); WANG, Jian, Hangzhou, Zhejiang 310052 (CN); ZHOU, Bin, Hangzhou, Zhejiang 310052 (CN)
(74) Representative: KIPA AB
(86) International application number: PCT/CN2021/121154
(87) International publication number: WO 2022/073441

(57) **Abstract**

Disclosed in the present application is a conveying system for an implantable medical device. The conveying system comprises a balloon catheter, and further comprises a sheath, an adjusting line and a locking line. The sheath is in sliding fit with the periphery of the balloon catheter and is used for wrapping the implantable medical apparatus; the adjusting line is used for fixing the implantable medical apparatus on the balloon catheter in a releasable manner, wherein one end of the adjusting line can be kept fixed to the balloon catheter, and the other end of the adjusting line can penetrate the implantable medical apparatus and is provided with a locking hole; and the locking line has an opposite locking state and unlocking state, wherein in the locking state, the locking line penetrates each locking hole to limit the implantable medical apparatus, and in the unlocking state, the locking line is separated from each locking hole to release the implantable medical apparatus. The present application effectively fixes the implantable medical apparatus to the conveying system so as to be conveyed to a lesion position, and adjusts or limits the relative position of the implantable medical apparatus in the axial direction of a balloon body by means of locking the locking line, the adjusting line and the implantable medical apparatus, and prevents accidental expansion of the implantable medical apparatus by means of wrapping of the controllable sliding sheath, thereby improving the operation safety thereof.

## Description

### TECHNICAL FIELD

The present application relates to the field of medical apparatus, in particular to a delivery system for implantable medical device and a control handle thereof, an implantable medical device and methods for securing, loading, and releasing the same.

### BACKGROUND

Invasive deployment of certain implantable medical devices has become the routine method for treating certain medical conditions. For example, stents and prosthetic heart valves are now delivered to treatment locations inside blood vessels and inside the heart via a transcatheter procedure that avoids invasive surgery.

To facilitate delivery to desired treatment locations, the implantable medical device (e.g., stents and prosthetic heart valves) is crimped on a balloon catheter, and then threaded through an introducer or a sheath which is inserted into the vasculature to the targeted location. The implantable medical device is then expanded by the balloon in the balloon catheter to its intended size and secured at the desired treatment location. The balloon is then deflated and the balloon catheter is withdrawn from the human body.

The securing of the implantable medical device on the balloon is normally accomplished by using friction, with shoulders provided on balloon catheter to block the implantable medical device from moving during the insertion and delivery. During the balloon inflation with gas / fluid, the balloon is normally first inflated at both ends thereof, so as to serve as a blockage to prevent the implantable medical device from moving laterally on the balloon.

While the current loading methods tend to be effective for most procedures, there can be some issues.

First, the crimped implantable medical devices are exposed to the surrounding environment (e.g., the traditional sheath, blood vessels, etc.), which may cause the implantable medical device to experience movement during insertion. When crossing the aortic arch, the exposed implantable medical device tends to flare at its distal end on the outer surface, which can be a hazard to the artery.

Second, the shoulders used in securing the implantable medical device on the balloon can hold the implantable medical device in place but carries a risk of piercing the balloon, as the implantable medical device tends to move and presses against the shoulders.

Thus, there remains a need for an improved delivery system or method for securing an implantable medical device to a delivery system for delivery to a treatment location, and for deployment of the implantable medical device at the treatment location, in a manner which avoids the drawbacks mentioned above.

### SUMMARY

In order to solve the above technical problems, the application discloses a delivery system for implantable medical device, including a balloon catheter, and further includes:
a sheath in sliding fit over the balloon catheter and for covering the implantable medical device;
an adjustment string for releasably securing the implantable medical device over the balloon catheter, one end of the adjustment string is capable of being fixed to the balloon catheter, and the other end is capable of passing through the implantable medical device and has an eyelet; and
a locking wire having relative locked and unlocked states, wherein in the locked state, the locking wire passes through the eyelet to restrain the implantable medical device, and in the unlocked state, the locking wire disengages from the eyelet to release the implantable medical device.

The following further provides several options, which are not used as additional limitations on the above-mentioned overall scheme, but only further additions or preferences. Without technical or logical contradiction, the options can be combined with the above-mentioned overall scheme, independently or in combination.

Optionally, the delivery system further comprises a control handle, to which all of the balloon catheter, the sheath and the locking wire extend, and the balloon catheter, the sheath, and the locking wire are movable relative to each other.

Optionally, the balloon catheter includes:
a guide head provided with a receiving hole, into which an end of the locking wire extends in the locked state;
a balloon adjacent to, and proximal to, the guide head, an outer periphery of the balloon being configured as a loading area for the implantable medical device; and
a catheter body communicated with the balloon and extending toward a proximal end of the delivery system.

Optionally, the balloon catheter further includes:
a central tubing passing through the catheter body, wherein one end of the central tubing extends toward the proximal end of the delivery system, and the other end passes through the balloon and is connected with the guide head.

Optionally, the locking wire is configured to extend from a lumen defined between the sheath and the catheter body toward the proximal end of the delivery system.

Optionally, the locking wire is configured to extend from a lumen defined in the catheter body toward the proximal end of the delivery system.

Optionally, a tube wall of the catheter body has a through hole, and a distal end of the locking wire is configured to extend outside the catheter body via the through hole to engage with the adjustment string.

Optionally, the tube wall of the catheter body has a radially concave area, and the through hole is opened in a proximal portion of the radially concave area.

Optionally, there is only one joint between the adjustment string and the locking wire.

Optionally, there are at least two joints between the adjustment string and the locking wire, and wherein at least one of the joints restricts movement of the implantable medical device in a distal direction, and at least another one restricts movement of the implantable medical device in a proximal direction.

Optionally, one or more adjustment strings are provided, and each adjustment string is provided with one or more eyelets.

Optionally, the eyelets are arranged in pairs, and in each pair, one of the eyelets is close to a distal end of the balloon catheter, and the other is close to a proximal end of the balloon catheter.

Optionally, the end of the adjustment string is coiled into a loop structure, and the interior of the loop structure is the eyelet.

Optionally, the adjustment string includes:
a first adjustment string having a first eyelet and connected to the balloon catheter and adjacent to a distal end of the balloon catheter; and
a second adjustment string having a second eyelet and connected to the balloon catheter and adjacent to a proximal end of the balloon catheter;
wherein in the locked state, the locking wire passes through the first eyelet and the second eyelet simultaneously.

Optionally, in the locked state, the distance between the end of the locking wire and the first eyelet is at least 5 mm, for example, ranging 8-30 mm.

Optionally, the first adjustment string and the second adjustment string are respectively fixedly connected to the balloon catheter.

Optionally, the first adjustment string is fixed to the guide head.

Optionally, the second adjustment string is fixedly provided on an outer periphery the catheter body.

Optionally, the outer periphery of the catheter body is provided with a limiting step, and the second adjustment string is limited by the limiting step.

Optionally, the first adjustment string and the second adjustment string are both movably connected to the balloon catheter.

Optionally, the first adjustment string and the second adjustment string are connected by a connecting string.

Optionally, at least one of the first adjustment string and the second adjustment string is formed with the connecting string in one piece.

Optionally, the connecting string extends toward the proximal end of the delivery system.

Optionally, the connecting string extends toward the proximal end of the delivery system through the central tubing and can be pulled relative to the balloon catheter.

Optionally, one of the first adjustment string and the second adjustment string is configured as a fixed adjustment string fixedly connected to the balloon catheter, and the other is configured as a movable adjustment string that can be pulled relative to the balloon catheter, and wherein a middle sheath is movably threaded through a lumen defined between the sheath and the catheter body, and the movable adjustment string is connected to the middle sheath.

Optionally, the central tubing has a plurality of lumens, through one of which the movable adjustment string extends.

Optionally, one of the plurality of lumens is a central lumen, and at least another one is an eccentrically defined second lumen, through which the movable adjustment string extends.

Optionally, two openings are defined in a lumen wall of the second lumen, and the first adjustment string and the second adjustment string extend out of the central tubing through the corresponding openings.

Optionally, the implantable medical device is provided with a ring, and in the locked state, an end of the adjustment string having the eyelet engages with the locking wire after passing through the corresponding ring.

Optionally, at least two rings are provided, which are provided at two opposite ends of the implantable medical device.

Optionally, the ring is formed in at least one of the following ways:
a, configured as in independent member and fixed to the implantable medical device;
b, opening a hole in the implantable medical device; and
c, directly using space of the implantable medical device itself.

Optionally, the implantable medical device has a tubular device body, and the end of the adjustment string having the eyelet is threaded from an interior of the device body radially towards an exterior of the device body.

Optionally, at least two rings are provided at the two axial ends of the device body.

Optionally, the locking wire is a metal rod.

Optionally, the delivery system further comprises a bending member which is located inside, outside, or sandwiched in a wall of the catheter body, and distal portions of the bending member and the catheter body are configured to interact with each other so as to bend a distal portion of the balloon catheter.

Optionally, a proximal end of the bending member is connected to and controlled by the control handle.

Optionally, the bending member is a bending wire or a bending tube, and wherein distal ends of the bending member and the catheter body are fixed with each, and proximal ends are in sliding fit so as to bend the distal portion of the balloon catheter.

Optionally, the bending member includes an inner bending tube and an outer bending tube surrounding the inner bending tube, the distal ends of the inner bending tube and the outer bending tube are fixed, and the proximal ends are in sliding fit so as to bend the distal portion of the bending member which, in turn, acts on the catheter body.

Optionally, the distal portions of the bending member and the catheter body are fixedly connected to or movably engaged with each other.

Optionally, at least one of the inner bending tube and the outer bending tube is movably connected to the control handle.

Optionally, the control handle for operating the implantable medical device comprises a support and a first driving assembly provided on the support, and wherein the first driving assembly comprises:
a first mounting base slidably arranged on the support; and
a first driving member movably arranged on the support and in transmission fit with the first mounting base; and
wherein the first mounting base comprises a main body with a lumen, and ports opened in the main body and communicated with the lumen:
   a distal port for docking with the balloon catheter;
   a driving port for injecting fluid into the balloon catheter;
   a limiting port for threading the locking wire; and
   a proximal port for threading the guide wire.

Optionally, the control handle further comprises a second driving assembly provided on the support, the second driving assembly is proximal to the first driving assembly, and the second driving assembly comprises:
a second mounting base slidably arranged on the support; and
a second driving member movably arranged on the support and in transmission fit with the second mounting base.

Optionally, the control handle further comprises a third driving assembly provided on the support, the third driving assembly is between the first and second driving assemblies, and the third driving assembly comprises:
a fixed mounting base;
a third mounting base slidably arranged on the support; and
a third driving member movably arranged on the support, and in transmission fit with the third mounting base.

Optionally, the support has an axial direction, the support is provided with guide grooves extending along the axial direction, and the mounting bases are slidably arranged in the corresponding guide grooves; and the driving members are respectively rotatably provided around the support, and in thread fit with the corresponding mounting bases.

Optionally, the support has an axial direction, the first mounting base is a four-way structure with four side tubes, and the distal port, the driving port, the limiting port and the proximal port are the corresponding tube openings of the respective side tubes; and

The distal port and the proximal port are aligned with each other in the axial direction of the support.

Optionally, axes of the four side tubes are substantially in the same plane.

Optionally, a proximal end of the catheter body is in sealing connection with the distal port, and a proximal end of the central tubing extends out of the catheter body and through the lumen of the first mounting base until in sealing connection with the proximal port; and

The driving port communicates to a radial gap between the catheter body and the central tubing.

Optionally, a proximal end of the locking wire is configured to extend into the lumen of the first mounting base, and then directly out of the first mounting base through the limiting port, or out of the first mounting base through a side wall of the side tube where the limiting port is located.

Optionally, the side tube where the limiting port is located extends obliquely toward a proximal end of the support with an angle α relative to an axis of the support satisfies 0 degree<α< 60 degrees. For example, 10 degrees<a<45 degrees, and for another example, 15 degrees<α<30 degrees.

The application discloses a delivery system for an implantable medical device, including a balloon catheter, and further includes:
a sheath in sliding fit over the balloon catheter and for covering the implantable medical device;
an adjustment string for releasably securing the implantable medical device over the balloon catheter, one end of the adjustment string is capable of being fixed to the balloon catheter, and the other end has an eyelet and is capable of passing through the implantable medical device; and
a locking wire having relative locked and unlocked states, wherein in the locked state, the locking wire passes through the implantable medical device to limit detachment of the implantable medical device from the adjustment string, and in the unlocked state, the locking wire is detached from the implantable medical device, allowing the eyelet to disengage from and release the implantable medical device.

Optionally, the implantable medical device is provided with a ring, and in the locked state, the ring passes through an end of the corresponding adjustment string having the eyelet and engages with the locking wire.

The application further discloses an implantable medical device, including a stent, and leaflets connected to the stent, wherein the stent is a radially deformable structure and has relative crimped and expanded states, and the implantable medical device further comprises a restraint ring connected to and around the stent, and the restraint ring is configured to restrain a radial deformation of the stent when the stent transforms into the expanded state.

Optionally, the restraint ring is arranged outside, inside, or undulating through the inside and outside of the stent.

Optionally, in an axial direction of the stent, the restraint ring is located at a middle of the stent or adjacent to an inflow side of the stent.

Optionally, in the axial direction of the stent, the restraint ring is located adjacent to an inflow side of the leaflets.

Optionally, in the axial direction of the stent, the restraint ring has a width of 2-30 mm, for example 5 to 20mm, for another example 5 to 15mm, and for another example, 8 mm.

Optionally, the restraint ring is made of a flexible material that can be compressed with the stent.

Optionally, the restraint ring is stitched to the stent.

Optionally, the material of the restraint ring is PTFE.

Optionally, the restraint ring is a metal wire.

Optionally, in an axial direction of the stent, the stent comprises a restrained section corresponding to the restraint ring and free sections, respectively, on two sides of the restrained section, and in the expanded state, the restrained section has a smaller outer diameter than the respective free sections.

Optionally, the stent is a mesh cylinder with a hollow structure and has an axial direction, depending on blood flow direction, one end of the stent in the axial direction is configured as an inflow side, the other end is configured as an outflow side, and an interior of the stent is configured as a blood flow channel extending in the axial direction, and 2, 3 or 4 leaflets are provided and cooperate with each other to open or close the blood flow channel.

Optionally, the implantable medical device further comprises a first covering film connected to an inside of the stent, and the first covering film is connected to the inflow side of the leaflets.

Optionally, the implantable medical device further comprises a second covering film connected to an outside of the stent, and the second covering film is connected on an inflow side of the restraint ring.

Optionally, the first covering film and the second covering film are formed in one piece.

Optionally, both the first covering film and the second covering film extend to the inflow side of the stent, and are connected with each other to cover an inflow side edge of the stent.

Optionally, one of the first covering film and the second covering film extends to the inflow side of the stent and folds back to cover an inflow side edge of the stent, and further extends to connect with an outflow side of the other covering film.

Optionally, the first covering film and/or the second covering film have cut areas at side edge thereof adjacent to the inflow side of the stent, and the cut areas are matched with the inflow side edge of the stent in shape.

Optionally, the inflow side of the stent comprises a plurality of cells arranged in a circumferential direction, with gaps located between adjacent cells and facing the inflow side, and the cut areas are matched with the corresponding gaps.

Optionally, the first covering film and/or the second covering film have a zigzag structure with teeth at the side edge thereof adjacent to the inflow side of the stent, and the cut area is located between adjacent teeth and two sides thereof are sewed and connected with the corresponding portions of the stent.

The present application provides an implantable system, including an implantable medical device and a delivery system matched with the implantable medical device.

The present application discloses a releasing method for implantable medical device which is preset in the delivery system. The releasing method includes:
driving the sheath toward a proximal end of the delivery system to expose the implantable medical device;
pulling the locking wire out from the eyelet of the adjustment string;
inflating the balloon to drive the implantable medical device to deform to an expanded state, and withdrawing the adjustment string out from the implantable medical device; and
deflating the balloon, and moving the balloon catheter toward the proximal end of the delivery system to separate from the implantable medical device.

The present application provides a loading method for implantable medical device for securing the implantable medical device to the delivery system, the loading method comprising:
placing the implantable medical device in an expanded state over a deflated balloon;
radially crimping the implantable medical device into a crimped state;
threading an end of the adjustment string with the eyelet through the implantable medical device;
threading the locking wire through the eyelet; and
sliding the sheath toward a distal end of the delivery system until it covers the implantable medical device.

The application provides a method for securing an implantable medical device over a balloon catheter, comprising the following steps:
providing the implantable medical device having a tubular device body that has a first end and a second end, with a first ring provided at the first end and a second ring provided at the second end;
providing a balloon catheter having:
   a catheter body that has a guide head,
   a balloon provided adjacent to, and proximal to, the guide head, and having a distal end and a proximal end,
   a first string having a first eyelet and secured to the balloon catheter at a position adjacent a distal end of the balloon catheter, and
   a second string having a second eyelet and secured to the balloon catheter at a position adjacent a proximal end of the balloon catheter;
providing a sheath for sliding movement over the catheter body and the balloon, the sheath having a distal end;
positioning the implantable medical device in its expanded state over the deflated balloon;

radially crimping the implantable medical device over the deflated balloon;
threading the first and second eyelets of the first and second strings, respectively, through the first and second rings, respectively;
advancing a locking wire through the first and second eyelets and into the guide head; and
   advancing the sheath over the crimped implantable medical device to the guide head to completely cover the crimped medical device;
   wherein the step of advancing a locking wire includes the step of advancing the locking wire through a lumen defined between the sheath and the catheter body to exit the distal end of the sheath.

Optionally, the step of threading the first and second eyelets includes the step of locking each of the first and second eyelets at the first and second rings, respectively.

Optionally, each of the first and second rings is provided as an independent ring and attached to the first and second ends, respectively, of the implantable medical device.

Optionally, each of the first and second rings is provided as a rounded tip at the first and second ends, respectively, of the implantable medical device.

Optionally, each of the first and second rings is provided as an opening in an apex at the first and second ends, respectively, of the implantable medical device.

Optionally, the method further includes providing a connecting wire that connects the first and second strings.

Optionally, the method further includes including providing a central lumen extending through the balloon, and wherein the connecting wire extends through the central lumen.

Optionally, the method further includes providing a middle sheath that extends through a lumen defined between the sheath and the catheter body, with the second string attached to a distal end of the middle sheath.

Optionally, a method for implanting an implantable medical device into a human anatomy, the implantable medical device has been secured to the deflated balloon of the balloon catheter, comprising the following steps:
withdrawing the sheath in a proximal direction to expose the implantable medical device;
withdrawing the locking wire in a proximal direction;
expanding the balloon, with the expanding balloon causing the first and second strings to be withdrawn through the first and second rings, respectively;
deflating the balloon; and
withdrawing the balloon catheter in a proximal direction.

In case where the implantable medical device is preferably an prosthetic heart valve, the present application accordingly provides an implantable prosthetic heart valve, a delivery system for an implantable prosthetic heart valve, and an implantable system for an prosthetic heart valve.

Similarly, a releasing method of the implantable prosthetic heart valve, a loading method of the implantable prosthetic heart valve and a method of securing the implantable prosthetic heart valve over the balloon catheter are provided.

The present application effectively secures the implantable medical device to the delivery system for delivery to the treatment location, and for deployment at the treatment location. The improved locking manner prevents the medical device from over the deflated balloon. In addition, providing the sheath prevents flaring of the ends of the medical device, thereby minimizing the risk of piercing the balloon.

### BRIEF DESCRIPTION OF THE DRAWINGS

FIG. 1A is an exploded side view illustrating an implantable medical device and a balloon catheter according to one embodiment of the present application;
FIG. 1B is a view that illustrates how the implantable medical device in FIG. 1A is positioned to surround the balloon;
FIG. 2 illustrates how the implantable medical device in FIG. 1A is crimped over the balloon;
FIG. 3A illustrates how strings are extended through the rings provided on the ends of the implantable medical device;
FIG. 3B illustrates how a locking wire is extended through eyelets on the adjustment strings and into a guide head;
FIG. 4 illustrates the sheath advanced over the implantable medical device;
FIG. 5 illustrates the withdrawal of the sheath to uncover the implantable medical device;
FIG. 6 illustrates the expansion of the balloon;
FIG. 7 illustrates the withdrawal of the locking wire from the eyelets and into the sheath;
FIG. 8 illustrates the deflation of the balloon into a deflated state;
FIG. 9 illustrates the withdrawal of the balloon catheter from the implantable medical device;
FIGS. 10-12B illustrate different embodiments for the implantable medical device in FIG. 1A with different rings;
FIG. 13A is a view illustrating an implantable medical device crimped over a balloon catheter according to another embodiment of the present application;
FIG. 13B is a cross-sectional side view of FIG. 13A;
FIG. 14A is a view of FIG. 13A showing the adjustment wire pulled taut;
FIG. 14B is a cross-sectional side view of FIG. 14A;
FIG. 15A is a view according to another embodiment of the present application configured with a middle sheath;
FIG. 15B is a view of FIG. 15A showing the locking wire pulled taut by the withdrawn middle sheath;
FIG. 16 is a schematic view of a delivery system according to an embodiment of the present application;
FIG. 17 is a schematic view showing how the rings at the ends of the implantable medical device passing through the eyelets of the adjustment strings;
FIG. 18 is a schematic view showing how the locking wire extends to the guide head after passing through the rings on the implantable medical device;
FIG. 19 is a schematic view showing the expanded balloon in FIG. 18;
FIG. 20 is a schematic view showing the connection between the bending member and the distal portion of the catheter body according to an embodiment of the present application;
FIG. 21 is an enlarged view of part K of FIG. 20 with the bending member and the catheter body connected in a fixed condition;
FIG. 22 is a schematic view showing how the moved bending member bends the catheter body of FIG. 21;
FIG. 23 is an enlarged view of part K of FIG. 20 with the bending member and the catheter body connected in a movable condition;
FIG. 24 is a schematic view showing how the moved bending member bends the catheter body of FIG. 23;
FIG. 25 is a schematic view of part of the balloon catheter according to an embodiment of the present application;
FIG. 26 is an enlarged view of part J in FIG. 25;
FIG. 27 is a schematic view showing the engagement between the second adjustment string and the limiting step of the catheter body according to an embodiment of the present application;
FIG. 28 is an exploded view of FIG. 16;
FIG. 29 is a sectional view of the control handle in FIG. 16;
FIG. 30 is a cross-sectional view of the balloon catheter in FIG. 16;
FIG. 31 is an enlarged view of part A in FIG. 28;
FIG. 32 is an enlarged view of part B in FIG. 28;
FIG. 33 is an enlarged view of part G in FIG. 29;
FIG. 34 is an enlarged view of part C in FIG. 28;
FIG. 35 is an enlarged view of part D in FIG. 28;
FIG. 36 is an enlarged view of part H in FIG. 29;
FIG. 37 is an enlarged view of part E in FIG. 28;
FIG. 38 is an enlarged view of part F in FIG. 28;
FIG. 39 is an enlarged view of part I in FIG. 29;
FIG. 40 is a partial enlarged view of the guide head in FIG. 30;
FIG. 41 is a schematic structural view of the first mounting base in FIG. 28;
FIG. 42 is a cross-sectional view of the first driving member in FIG. 16;
FIG. 43 is a schematic view of an implantable medical device in a crimped state after being loaded with a restraint ring according to an embodiment of the present application;
FIG. 44 is a schematic view of an implantable medical device in an expanded state after being loaded with a restraint ring according to an embodiment of the present application;
FIG. 45 is a perspective view of an implantable medical device in an expanded state after being loaded with a restraint ring according to an embodiment of the present application;
FIG. 46 is a schematic view of part of a first covering film for an implantable medical device according to an embodiment of the present application;
FIG. 47 is a schematic view of part of the first covering film and the second covering film for an implantable medical device according to an embodiment of the present application;
FIG. 48 is a schematic view of part of the first covering film and the second covering film for an implantable medical device according to another embodiment of the present application; and
FIG. 49 is a schematic structural view of the exhaust assembly of the control handle according to an embodiment of the present application.

The reference numbers in the figures are listed as follows:
100, implantable medical device; 102, cell; 103, stent; 104, heart valve assembly; 105, ring; 105a, ring; 105b, apex; 105c, apex; 105d, ring; 105e, ring; 106, distal end; 107, proximal end; 108, leaflet; 109, restraint ring; 111, first covering film; 112, second covering film; 113, inflow side; 114, outflow side; 115, hole;
120, balloon catheter; 121, first adjustment string; 122, balloon; 123, sheath; 124, catheter body; 126, locking wire; 127, guide head; 128, proximal end; 129, connector; 131, second adjustment string; 132, distal end; 133, central tubing; 135, first eyelet; 136, second eyelet; 137, lumen; 138, receiving hole; 139, limiting step;
140, connecting string; 8a, locking area; 8b, locking area; LK, locking area; 11a, locking knot; 11b, locking knot; 141, central lumen; 142, second lumen; 143, second opening; 144, first opening; 150, the middle sheath;
160, concave area; 161, through hole;
200, control handle; 210, support; 211, guide groove; 212, guide surface; 220, first driving assembly; 221, first mounting base; 222, first driving member; 231, second mounting base; 232, second driving member; 240, exhaust assembly; 241, connector; 242, liquid injection pipe; 250, third driving assembly; 251, third mounting base; 252, third driving member; 253, fixed mounting base; 261, distal port; 262, driving port; 263, limiting port; 264, proximal port; 265, operating member;
300, bending member; 301, inner bending tube; 302, outer bending tube.

### DESCRIPTION OF THE EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this invention.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes one or more of the listed options in any combinations, or the combination of all of the listed options.

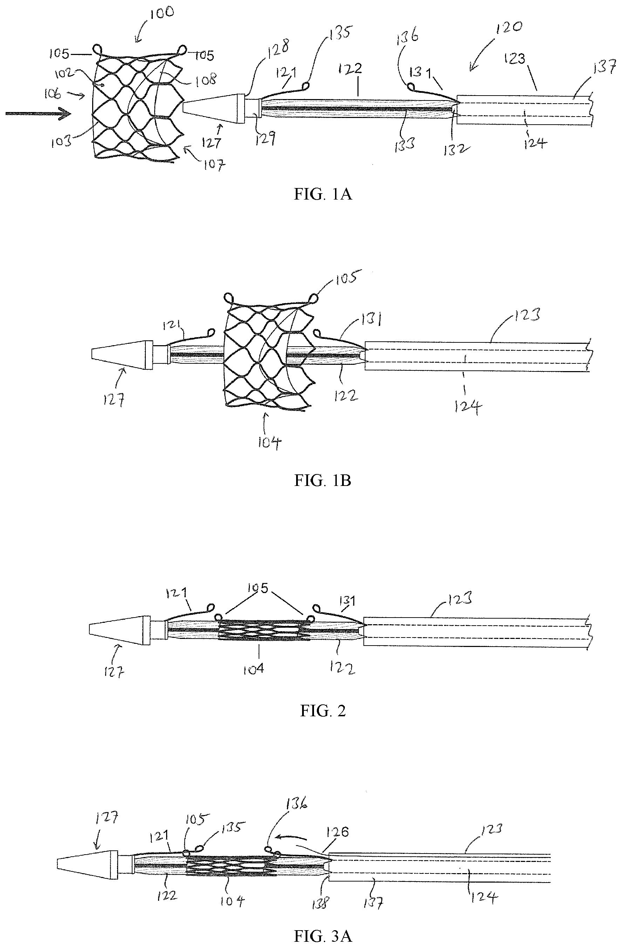

Please refer to FIG. 1A, the present application provides a delivery system for an implantable medical device, and the implantable medical device 100 can be any implantable medical device that is intended to be implanted into the human body, such as a stent, transcatheter heart valve, stent-graft assembly, closure device, and plug, among others. The drawings in the present application are illustrated in connection with a transcatheter heart valve assembly 104 which can be an expandable stent, or a stent-frame for use with a transcatheter heart valve. Taking the heart valve as an example, it can be applied to the aortic valve, mitral valve, tricuspid valve, pulmonary valve or the like.

The heart valve assembly 104 has a tubular device body, for example, a hollow cylindrical stent 103. The stent 103 has a strut structure formed by weaving or cutting, for example, it can be woven from a single wire or multiple wires, or laser cut from a metal (e.g., nickel-titanium) tube. The strut structure defines a plurality of cells 102.

Referring to FIG. 1A to FIG. 15B, the delivery system for the implantable medical device of the present application includes a balloon catheter 120, and further includes:
a sheath 123 slidably engaged over the balloon catheter 120 for covering the implantable medical device 100;
an adjustment string for releasably securing the implantable medical device 100 over the balloon catheter 120, wherein one end of the adjustment string can be fixed with the balloon catheter 120, and the other end can pass through the implantable medical device 100 and has an eyelet; and
a locking wire 126 having a relative locked state and an unlocked state, wherein in the locked state, the locking wire 126 passes through the corresponding eyelets to restrain the implantable medical device, and in the unlocked state, the locking wire 126 falls off from the corresponding eyelets to release the medical device 100.

The balloon catheter 120 has an axial length and has a corresponding proximal end 132 and a distal end 128. Unless otherwise specified below, the distal end 128 is the end where the balloon catheter 120 is first inserted into the human body, and the proximal end 132 is the end opposite to the distal end 128. The delivery system has proximal and distal ends in the same orientation as the balloon catheter 120. That is, the other components of the delivery system, such as the sheath 123, locking wire 126, etc., have the same distal end 128 and proximal end 132 as the balloon catheter 120.

The implantable medical device 100 can be detached from the delivery system. For the convenience of description, the implantable medical device 100 has a distal end 106 and a proximal end 107.

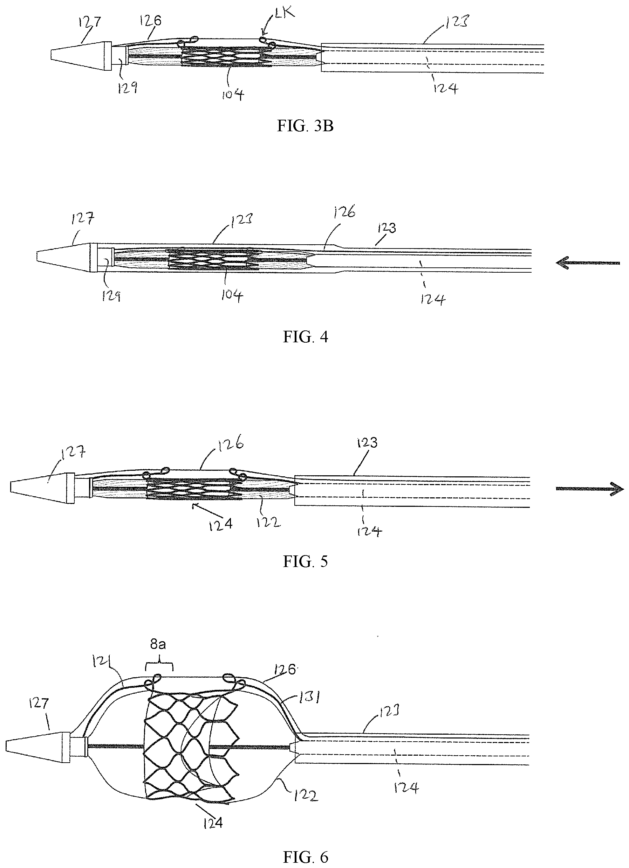

The balloon catheter 120 is made of elastic material and disposed at the distal end of the delivery system, and is bendable so that the implantable medical device 100 disposed thereon can be delivered to the lesion. Referring to FIG. 1B and FIG. 6, the balloon catheter 120 can be inflated with gas / fluid to expand itself, driving the implantable medical device 100 to an expanded state. After discharging the gas / fluid, the balloon catheter 120 assumes a deflated state.

The implantable medical device 100 has a crimped state and an expanded state, and is switchable between the expanded and crimped states.

Referring to FIG. 4, the sheath 123 is a hollow tube, which is slidably provided within the delivery system and configured to slide axially along the balloon catheter, thereby changing the restraint on the implantable medical device 100. When the sheath 123 surrounds the balloon catheter 120, it can restrain the implantable medical device 100 in the interior thereof, thereby limiting the expansion of the implantable medical device 100. When the sheath 123 slides relative to the balloon catheter 120 and completely offsets from the expansion path of the implantable medical device 100 to expose the implantable medical device 100, the implantable medical device 100 would be allowed to expand.

The adjustment string is used to avoid the misalignment between the implantable medical device 100 and the balloon catheter 120 (especially in the axial direction of the balloon catheter 120) during the procedure, which may affect the three-dimensional profile of the implantable medical device 100 after it enters the expanded state, or even pose a safety hazard. Provided that the axial misalignment is avoided to the maximum extent, the adjustment string releasably securing the implantable medical device 100 on the balloon catheter 120 should not affect the release of the implantable medical device 100 either.

In order to ensure the restraint, one end of the adjustment string can be fixed to the balloon catheter 120. In some other cases, this end can be controlled to move relative to the balloon catheter 120, so that the position of the implantable medical device 100 can be flexibly adjusted, and interference on the release of the implantable medical device 100 can be avoided.

The controllable movement can be achieved by means of the elastic deformation of the adjustment string itself within a desired extent, or by operating the adjustment string through transmission components, or the like.

The other end of the adjustment string passes through the implantable medical device 100 and is restrained by the locking wire 126. The specific threading way of the adjustment string through the implantable medical device 100 is not strictly limited. The joint of the eyelet of the adjustment string and the locking wire can be considered as a limiting point. At a certain limiting point, at least the movement of the implantable medical device in a certain direction along the axial direction of the balloon catheter can be limited or interfered, so as to reduce the risk of misalignment therebetween.

The main function of the locking wire is to maintain the threading of the adjustment string through the implantable medical device, and to prevent the adjustment string from detaching from the implantable medical device. The locking wire itself can have a proper rigidity so that it can locally bear the shearing force from the eyelet of the adjustment string.

In one embodiment where there is only one joint between the adjustment string and the locking wire, if the adjustment string itself is a rigid member, even one joint can limit the misalignment of the implantable medical device in two directions. If the adjustment string is flexible and the length of the deformable part is short, one joint can also generally achieve the same effect.

In another embodiment where there are at least two joints between the adjustment string and the locking wire, at least one joint can limit the movement of the implantable medical device in the distal direction, and at least another joint can limit the movement of the implantable medical device in the proximal direction.

Alternatively, if only misalignment limit in a certain direction is desired, the above configuration can be further simplified.

One or more adjustment strings can be provided, and each adjustment string can be provided with one or more eyelets. In one embodiment, the eyelets can be arranged in pairs, with one or more pairs, for example. In each pair of eyelets, one eyelet is close to the distal end of the balloon catheter (in use state), and the other is close to the proximal end of the balloon catheter.

When the adjustment string restrains the implantable medical device, mutual pull between the adjustment string as a whole (if multiple adjustment strings are provided, consider the multiple adjustment strings as a whole) and the balloon catheter occurs in two directions.

The joint of the eyelet and the locking wire adjacent to the distal end of the balloon catheter can limit the movement of the implantable medical device to the proximal end;

The joint of the eyelet and the locking wire adjacent to the proximal end of the balloon catheter can limit the movement of the implantable medical device to the distal end.

The detachment between the locking wire and the adjustment string can be actively controlled. Alternatively, the locking wire can be automatically detached from the adjustment string by means of the deformation of the balloon catheter and implantable medical device at a specific step of the procedure.

In this embodiment, the implantable medical device 100 is provided with a ring 105. In the locked state, the ring 105 passes through the end of the corresponding adjustment string with the eyelet, and engages with the locking wire 126.

After the end of the adjustment string with the eyelet passes through the ring 105, the adjustment string is prevented from disengaging from the ring 105 by the locking wire 126, so that the adjustment string cannot be detached from the implantable medical device 100. Therefore, when the implantable medical device 100 slides over the balloon catheter 120, it would be eventually prevented from further sliding at the eyelet, limiting the axial sliding of the implantable medical device 100 in the crimped/expanded state relative to the balloon catheter. Alternatively, it may be the ring 105 that passes through the eyelet, and then the ring 105 is prevented from disengaging from the adjustment string by the locking wire 126.

The threading of the eyelet through the ring 105 or the ring 105 through the eyelet, and the locking wire through the eyelet or the ring 105 can be done manually or in other ways.

The locking wire 126 is movably provided within the delivery system. After passing through all the eyelets or the rings 105 in sequence, the two ends of the locking wire 126 are fixed in the locked state, and the interaction among the three prevents the adjustment string from detaching from the implantable medical device 100. The locking wire 126 is detachable from the delivery system at its distal end. After the locking wire 126 is withdrawn and disengaged from all the eyelets or the rings 105, it enters the unlocked state.

In one embodiment, the delivery system further includes a control handle 200, the balloon catheter 120, the sheath 123 and the locking wire 126 all extend to the control handle 200, and the balloon catheter 120, the sheath 123, and the locking wire 126 can move relative to each other.

Alternatively, the balloon catheter 120, the sheath 123, and the locking wire 126 can be movable relative to the control handle 200, and the distal movement can be controlled through the proximal control handle 200 to which the catheter and the wire extend. The movement of the balloon catheter 120 can realize the adjustment of itself and the implantable medical device at the lesion site, the sheath 123 can slide along the axial direction of the balloon catheter to cover or expose the implantable medical device 100, and the movement of the locking wire 126 can realize the detachment from the adjustment string.

Referring to FIGS. 1A to 3B, in some embodiments, the balloon catheter 120 includes :
a guide head 127 provided with a receiving hole 138, wherein the end of the locking wire 126 in the locked state extends into the receiving hole;
a balloon 122 provided adjacent to and proximal to the guide head 127, wherein the periphery of the balloon 122 is the loading area for the implantable medical device 100; and
a catheter body 124 communicated with the balloon 122 and extending toward the proximal end of the delivery system.

The distal end of the balloon catheter 120 is the guide head 127, and the locking wire 126 is configured to be inserted into the receiving hole and fixed therein, so as to avoid unintended release of the adjustment string.

The balloon 122 is disposed adjacent to and proximal to the guide head 127. The guide head 127 can have a tapered configuration from its proximal end 128 to its distal-most end which facilitates the delivery of the proximal balloon 122 and catheter body 124 in the human body, and a cylindrical connector 129 having a smaller diameter than that of the proximal end 128 can be provided at the proximal end 128 and extend in the proximal direction.

The distal end of balloon 122 is secured to the proximal end surface of connector 129 and the proximal end of the balloon 122 is secured to distal end 132 of catheter body 124. The balloon 122 is hollow and can be inflated with gas / fluid, through which other members can pass.

The catheter body 124 is a hollow tube, and the proximal end thereof extends into the control handle 200. In addition to accommodating catheter and wire for distal control, the interior of the catheter body can also be served as a fluid channel communicating with the interior of the balloon for delivering gas / fluid to inflate the balloon 122. The sheath 123 is slidable along the length direction of the catheter body 124 over the peripheries of the catheter body 124 and the balloon 122, functioning as a protector. Moreover, both the balloon 122 and the catheter body 124 are made of elastic material, facilitating the deformation.

Referring to FIG. 1A, in one embodiment, the balloon catheter 120 further includes:
a central tubing 133 extending through the catheter body 124, wherein one end of the central tubing 133 extends toward the proximal end of the delivery system and is connected to the control handle 200, and the other end passes through the balloon 122 and is connected to the guide head 127.

The central tubing 133 is a hollow tube made of elastic material and is bendable. Two ends of the central tubing 133 are communicated with each other, and the interior thereof is used as a guide wire channel.

The materials of the above-mentioned members are all explained here. The catheter body 124 can be made of Pebax, PTFE, Nylon, or any other known material that is used for catheter bodies; the sheath 123 can be made of Pebax, PTFE, Nylon or any other known material that is used for similar slidable sheaths; the adjustment string can be made of polypropylene suture, braided PET suture, PTFE sutures, or any conventional suture. Balloon 122 can be made of any conventional balloon material, such as Nylon, and Pebax^{™}.

Please refer to FIG. 1A to FIG. 7, the locking wire 126 can be arranged in any of the following ways:
In one embodiment, the locking wire 126 extends from a lumen 137 defined between the sheath 123 and the catheter body 124 toward the proximal end of the delivery system.

The sheath 123 is slidably disposed around the catheter body 124, and the lumen 1 37 for accommodating the locking wire 126 is defined therebetween.

In another embodiment, the locking wire 126 extends from a lumen 137 defined inside the catheter body 124 toward the proximal end of the delivery system.

In another embodiment, the wall of the catheter body 124 is provided with a through hole 161 through which the distal end of the locking wire 126 extends to the outside of the catheter body 124 for engaging with the adjustment string. In this embodiment, the wall of the catheter body 124 has a radially concave area 160, and the through hole 161 is opened in the proximal portion of the radially concave area 160.

The concave area 160 reduces the bending extent of the locking wire 126 at the through hole 161, so that the locking wire 126 can move more smoothly. In other embodiments, the through hole is an elongated hole, and the length direction thereof is in line with the axis of the catheter body.

In one embodiment, the locking wire is a metal rod.

The locking wire 126 can be made of 304 or 316 stainless steel (the diameter of the locking wire can generally be about 0.5 mm), so that a strong axial pushing force can be applied thereon, facilitating the threading through the eyelets or the rings. The outer surface of the locking wire 126 is coated with PTFE, reducing the friction with other tubes and wires; and since the locking wire 126 is not sticky, so that it can slide smoothly.

In one embodiment, the end of the adjustment string is coiled into a string loop structure, and the interior of the string loop structure is the eyelet.

The eyelet can also be provided by another string coiled to form a loop structure and then fixed with the end of the adjustment string. The string material can be the same as the adjustment string material or other materials. Of course, the provision of the eyelet is not limited to this.

The eyelet is generally a closed structure in the circumferential direction, but it can alternatively use a non-closed structure in the circumferential direction provided that the locking wire can be prevented from falling off therefrom.

In one embodiment, the adjustment string includes:
a first adjustment string 121 having a first eyelet 135, the first adjustment string 121 is connected to the balloon catheter 120 and adjacent to the distal end 106 of the balloon catheter 120; and
a second adjustment string 131 having a second eyelet 136, the second adjustment string 131 is connected to the balloon catheter 120 and adjacent to the proximal end 107 of the balloon catheter 120.

In the locked state, the locking wire 126 passes through the first eyelet 135 and the second eyelet 136 simultaneously.

The specific connection of the corresponding two ends of the two adjustment strings refer to the above description. Moreover, the two adjustment strings are located at the proximal end and the distal end of the balloon catheter 120 respectively, so as to restrain the implantable medical device 100 in two axial directions.

The first adjustment string 121 and the second adjustment string 131 can include one or more strings respectively. In case of multiple strings, the strings are provided in spaced apart manner along the circumferential direction of the balloon catheter 120. The two adjustment strings can be connected to the balloon catheter 120 and aligned or misaligned with each other in the circumferential direction of the balloon catheter 120.

One or more locking wires can be provided. Taking the first adjustment string 121 as an example, if the number of locking wires is less than that of the first adjustment strings 121, the first eyelets 135 of the first adjustment strings 121 can share one of the locking lines. In case of multiple locking wires, the locking wires can be independently controlled or linked with each other, preferably taking synchronous movement.

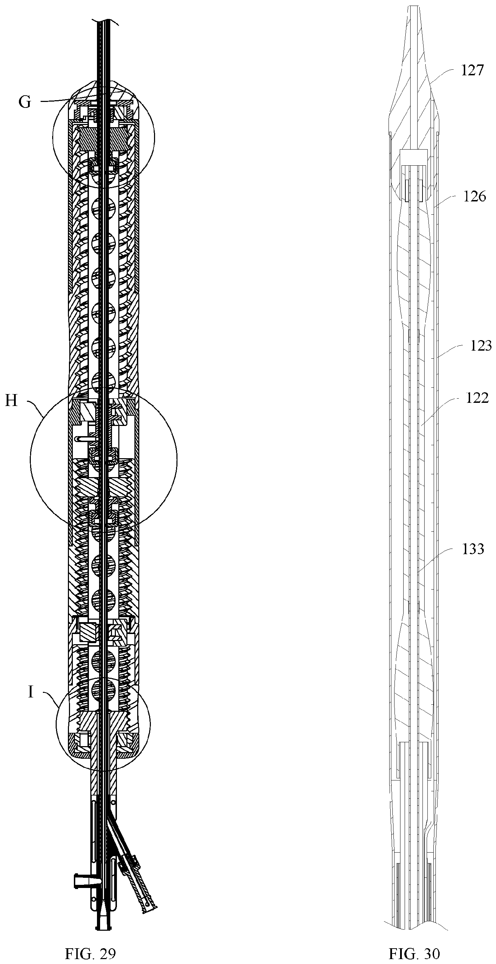

The motion range of the implantable medical device 100 over the balloon catheter 120 depends on the protruding distance of the first eyelet 135 and the second eyelet 136 relative to the implantable medical device 100. As shown in FIGS. 3B, 30 and 40, the locking wire 126 is advanced through the first eyelet 135 and the second eyelet 136 and into the receiving hole 138 inside the guide head 127. The locking location LK in FIG. 3B illustrates a locking knot where the locking wire 126 passes through the eyelets sequentially after the eyelets have been threaded through the corresponding rings 105. The hole inside the guide head 127 should have a certain depth so that the locking wire 126 can be securely locked inside the guide head 127. In this embodiment, the heart valve assembly 104 is securely restrained over the balloon 122 from any lateral movement, with the restraint provided by the first and second strings 121 and 132 and the locking wire 126.

In the locked state, the distance between the end of the locking wire 126 and the first eyelet 135 satisfies at least 5 mm, for example, 8-30 mm.

In some embodiments, the adjustment strings can be connected to the balloon catheter using different configurations.

In one embodiment, one of the first adjustment string 121 and the second adjustment string 131 is a fixed adjustment string fixedly connected to the balloon catheter 120, and the other one can be a movable adjustment string that can be pulled and moved relative to the balloon catheter 120.

In one embodiment, the first adjustment string 121 and the second adjustment string 131 are respectively fixedly connected to the balloon catheter 120.

The first adjustment string 121 can be directly fixed to at least one of the guide head 127, the connector 129 or the balloon, and the specific fixing method can be at least one of binding, bonding and the like.

For example, the end of the adjustment string can be bound to the above-mentioned corresponding member (at least one of the guide head, connector or balloon). In order to ensure the connection strength, at least one of the following methods can be further used:
coiling the end of the adjustment string into a string loop;
opening a through hole inside the corresponding member for the adjustment string to pass through; and
hot-melt bonding the end of the adjustment string with the corresponding member.

In one embodiment, the first adjustment string 121 is fixed to the connector 129, and the first adjustment string 121 extends from the connector 129 toward the proximal end of the balloon 122. Similarly, the second adjustment string 131 is fixed to the distal end 132 of the catheter body 124 and has an eyelet, and the second adjustment string 131 extends from the distal end 132 toward the distal end of the balloon 122.

In another embodiment, the first adjustment string 121 is fixed to the interior of the guide head 127.

The second adjustment string 131 can be directly fixed to at least one of the catheter body or the balloon, and the specific fixing method refers to the connection method of the first adjustment string 121.

In one embodiment, the second adjustment string 131 is fixedly provided on the outer periphery of the catheter body 124.

For example, the outer periphery of the catheter body 124 can be provided with a limiting step 139, and the second adjustment string 131 is limited by the limiting step.

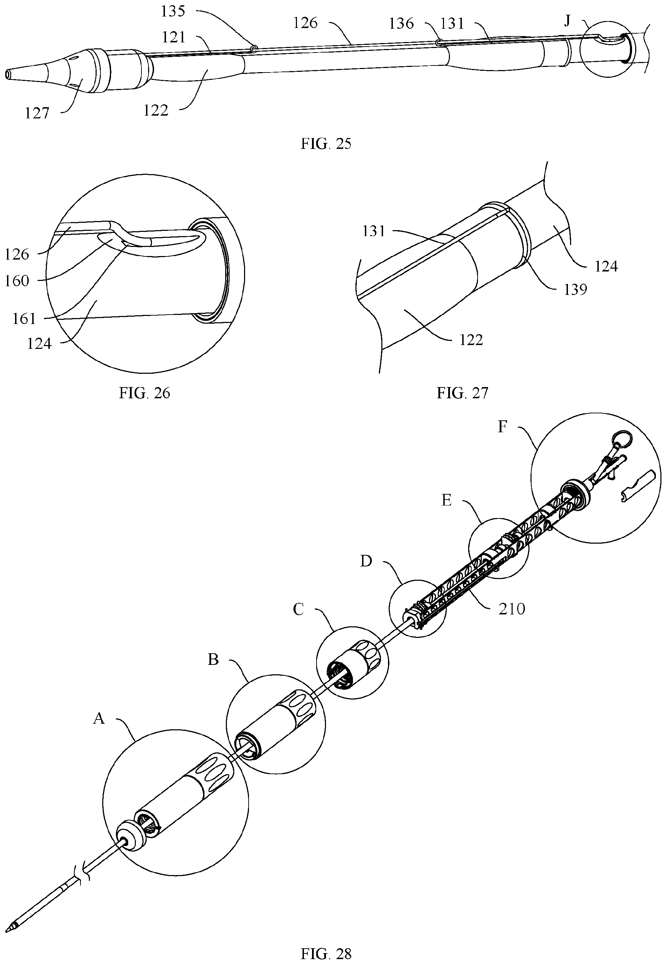

Referring to FIG. 27, the end of the second adjustment string 131 for connecting with the catheter body 124 is bound to form a loop structure located around the catheter body 124 and at the proximal end of the limiting step 139. Alternatively, a limiting groove can be used for receiving the coiled end of the second adjustment string 131.

In another embodiment, both the first adjustment string 121 and the second adjustment string 131 are movably connected to the balloon catheter 120.

One end of each adjustment string has an eyelet, and the other end can be considered as a movable end so that it can be controllably moved back and forth relative to the balloon catheter 120 so as to control the length of the exposed part of the adjustment string outside the balloon catheter 120, thereby limiting the sliding range of the implantable medical device 100.

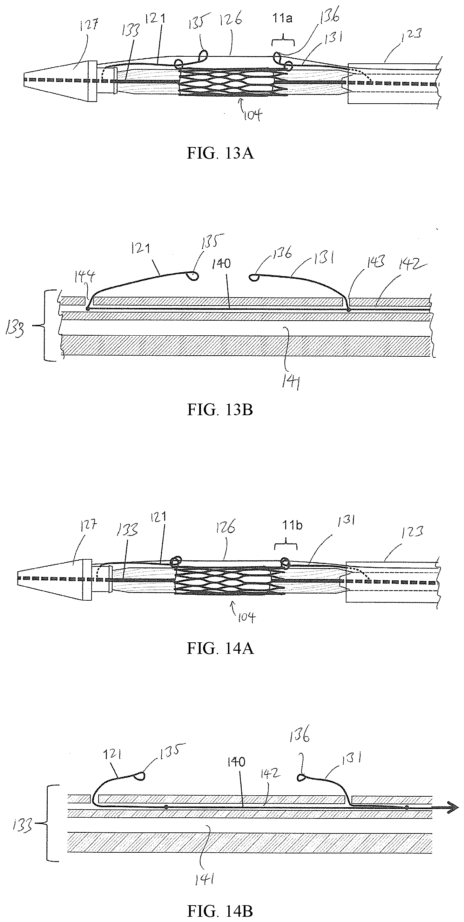

Referring to FIG. 14B, in one embodiment, the first adjustment string 121 and the second adjustment string 131 are connected through a connecting string 140. By controlling the connecting string 140, the first adjustment string 121 and the second adjustment string 131 can be driven simultaneously.

FIGS. 13A and 13B show the connecting string 140 in a relaxed state, so that the exposed parts of the first adjustment string 121 and the second adjustment string 131 can be longer. As shown in FIG. 13A, when the exposed parts of the first and second strings 121 and 131 are longer, the crimped implantable heart valve assembly 100 has room to slide back and forth (see the adjustable locking knot 11a) and is therefore poorly secured. When the connecting string 140 is pulled taut, the first and second strings 121 and 131 are also pulled along, so that the exposed parts of the first and second strings 121 and 131 become shorter. As shown in FIG. 14A, the first and second eyelets 135 and 136 at the ends of the first and second strings 121 and 131, respectively, are aligned with the rings 105 on the implantable heart valve assembly 100 (see the adjustable locking knot 11b), so that the implantable heart valve assembly 100 is tightly secured.

Preferably, at least one of the first adjustment string 121 and the second adjustment string 131 is provided with the connecting string 140 in one piece.

The connection of two strings (such as by bonding or binding) will thicken the joint. Using one piece structure will reduce the joint.

In one embodiment, the connecting string 140 extends towards the proximal end of the delivery system.

The connecting string 140 extends to the proximal control handle 200 for easy control.

The threading path of the connecting string 140 is not strictly limited. For example, it can extend inside the central tubing, outside the catheter body, between the central tubing and the catheter body, in the wall of the central tubing, or in the wall of the catheter body.

In one embodiment, the connecting string 140 extends inside the central tubing 133 toward the proximal end of the delivery system, and can be pulled and moved relative to the balloon catheter 120.

For example, when the connecting string 140 is moved proximally, the exposed parts of the two adjustment strings are shortened, limiting the relative movement of the implantable medical device 100. When releasing the implantable medical device 100, the connecting string 140 can be moved slightly to the distal end in order to adapt to the size change of the implantable medical device 100.

Similarly, when the two adjustment strings are fixedly provided, after passing through the implantable medical device 100, the two adjustment strings still have proper room to adapt to the release of the implantable medical device 100. Of course, the room should at least prevent the implantable medical device 100 from excessive misalignment. If the axial length of the balloon catheter 120 is sufficient, the room can be larger.

As shown in FIGS. 13A-14B, for the convenience of threading the wire or strings (such as guide wire, connecting string, and adjustment string), in one embodiment, the central tubing 133 has multiple lumens, wherein the movable adjustment string extends within one of the lumens, and the other lumens can receive guide wire or other string, so that the strings and guide wire each have a separate lumen, without interfere with each other and avoiding winding interference.

In this embodiment, among the lumens, one of them is the central lumen 141, and at least another lumen is an eccentrically defined second lumen 142, through which the movable adjustment string extends. In use, the guide wire extends through the central lumen 141.

In one embodiment, the wall of the second lumen 142 is provided with two openings, and the first adjustment string 121 and the second adjustment string 131 extend out of the central tubing 133 through the corresponding openings.

The two openings are provided in spaced apart manner, and are respectively the first opening 144 at the distal end and the second opening 143 at the proximal end. The two openings communicate the second lumen 142 with the exterior of the central tubing 133, allowing the two adjustment strings to protrude from and expose out of the central tubing 133. The first adjustment string 121 extends through the first opening 144, and the second adjustment string 131 extends through the second opening 143. The connecting string 140 extends through the second lumen 142 between the two openings such that a first end of the connecting string 140 connects to the first adjustment string 121, and the opposing second end of the connecting string 140 connects to the second adjustment string 131.

In one embodiment, a middle sheath 150 is movably provided in the lumen defined between the sheath 123 and the catheter body 124, and the movable adjustment string is connected to the middle sheath 150. The middle sheath 150 serves as a transmission member, which can extend proximally more conveniently and be connected with the control handle. The second adjustment string 131 can be fixed to the distal end of the middle sheath 150, while the first adjustment string 121 can be secured directly to, or adjacent to, the connector 129.

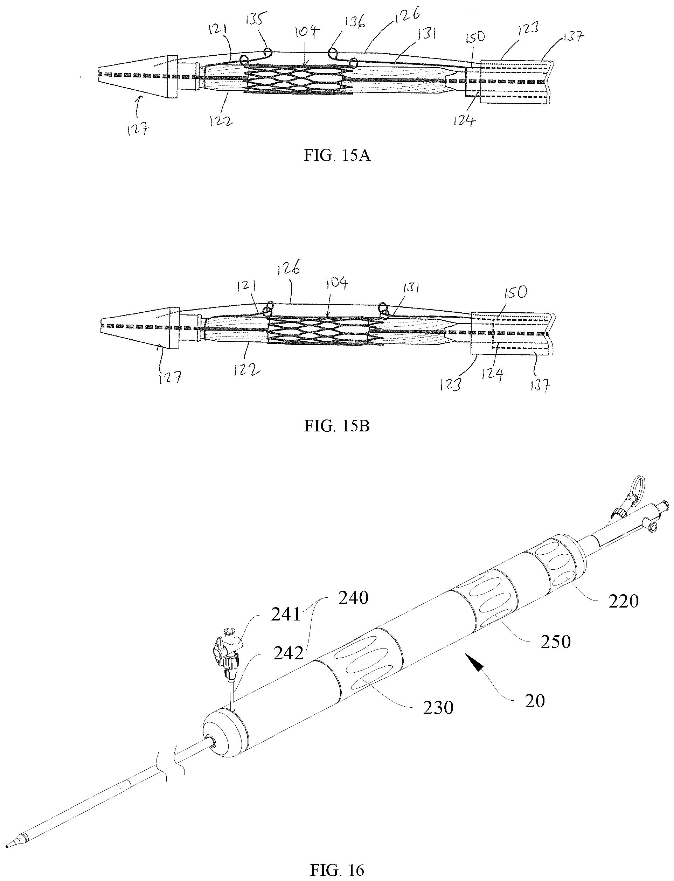

In FIG. 15A, the middle sheath 150 is advanced in a distal direction towards the balloon 122, with the distal end of the middle sheath 150 exposed by the sheath 123, so that the first and second eyelets 135 and 136 can be extended through and past the corresponding rings 105.

In FIG. 15B, the middle sheath 150 is then advanced in a proximal direction away from the balloon 122, which causes the first and second strings 121 and 131 to be pulled taut, and the first and second eyelets 135 and 136 to be locked with the corresponding rings 105. This embodiment (FIGS. 15A and 15B) operates under similar principles as the embodiments above, except that the middle sheath 150 is provided instead of a longer adjustment string or connecting string.

In summary, in one embodiment, there are at least two rings 105 on the implantable medical device, at least one of which is arranged adjacent to the opposite end of the implantable medical device 100. Alternatively, the at least two rings 105 are located at the two opposite ends of the implantable medical device 100 respectively, for example, the two opposite axial ends of the implantable medical device.

The two rings 105 are respectively used for the first eyelet 135 and the second eyelet 136 to pass through, and after the locking wire 126 passing through the two eyelets in turn, the end of the locking wire 126 is then fixed relative to the guide head, thereby completing the locking.

Alternatively, the two rings 105 can pass through the first eyelet 135 and the second eyelet 136, respectively, and after the locking wire 126 passing through the two rings 105 in turn, the end of the locking wire 126 is then fixed relative to the guide head, thereby completing the locking.

The rings can be aligned or misaligned with each other in the circumferential direction of the implantable medical device. The alignment means that the imaginary line connecting the two rings 105 is parallel to the axis of the implantable medical device (the implantable medical device is approximately configured as a straight cylinder), and the misalignment means the imaginary line connecting the two rings 105 is un-parallel to the axis of the implantable medical device.

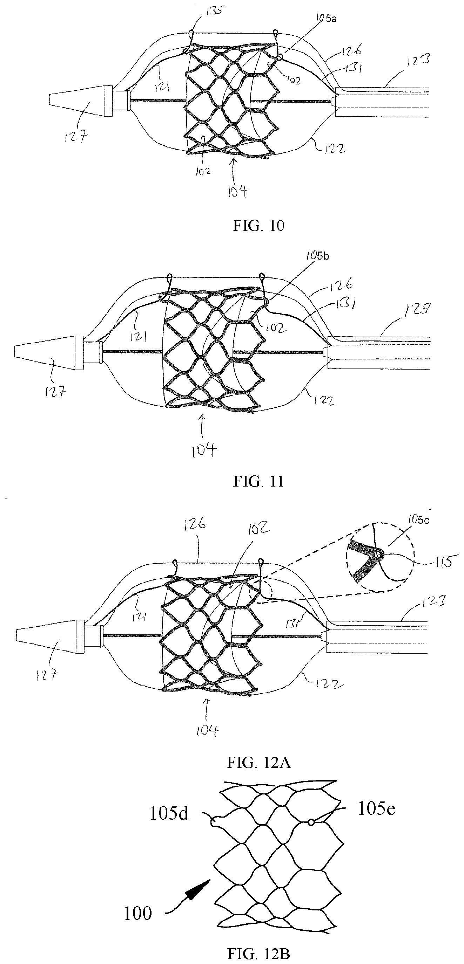

In some embodiments, the rings 105 are provided in at least one of the following methods:
Method a: the rings are independent rings and fixed to the implantable medical device 100, as shown in FIG. 10, the ring 105a can be made of the same or different material from the material of the implantable medical device 100. During the assembly, the rings 105a are manually attached to the stent 103. The rings 105a can be made of any conventional suture material (e.g., polypropylene, PTFE) or the same material as the stent 103.
Method b: the rings are provided by opening holes in the implantable medical device 100.

FIG. 12A shows holes 115 provided in the apices 105c of the cells 102, with the first and second strings 121, 131 adapted to extend into the holes 115 or conversely.

Method c: the rings are provided by using the space of the implantable medical device 100.

FIG. 11 shows another alternative where the rings 105 are apices 105b of the cells 102. The ring 105 is surrounded by the struts surrounding the cell. The apices 105b are arc-shaped to avoid cutting the adjustment string. The first adjustment string 121 and the second adjustment string 131 are adapted to extend into the space defined by the apices 105b in the same manner they would extend into the openings of the rings 105. The side of the apex 105b towards the inside of the cell is the open side, which facilitates the threading of the adjustment string into the ring 105.

A single implantable medical device 100 can have multiple forms of rings 105. For example, as shown in FIG. 12B, the implantable medical device 100 is provided with a pair of rings 105 which are aligned with each other in the circumferential direction of the implantable medical device 100, i.e., the rings 105d and 105e, wherein the ring 105d is provided by using the space of the implantable medical device 100, while the ring 105e is formed by opening a hole in the implantable medical device 100. The axial position of the rings on the implantable medical device 100 can be various. For example, the ring 105d can be provided at one axial end of the implantable medical device 100, while the ring 105e can be provided adjacent to, instead of right at one axial end of the implantable medical device 100.

In one embodiment, the implantable medical device 100 has a tubular device body, and one end of the adjustment string with an eyelet is threaded from the interior of the device body radially toward the exterior of the device body.

The interior of the device body faces the balloon catheter 120, and the exterior of the device body faces away from the balloon catheter.

The eyelet passes through the corresponding ring 105 from the inside to the outside, and is eventually located at the outside of the implantable medical device 100. The locking wire 126 passes through the eyelets in turn on the outside of the device body. For example, the threading direction of the locking wire 126 can be in line with the axial direction of the balloon catheter 120. The locking wire 126 can be threaded on the outside without obstacles, which is convenient for assembly.

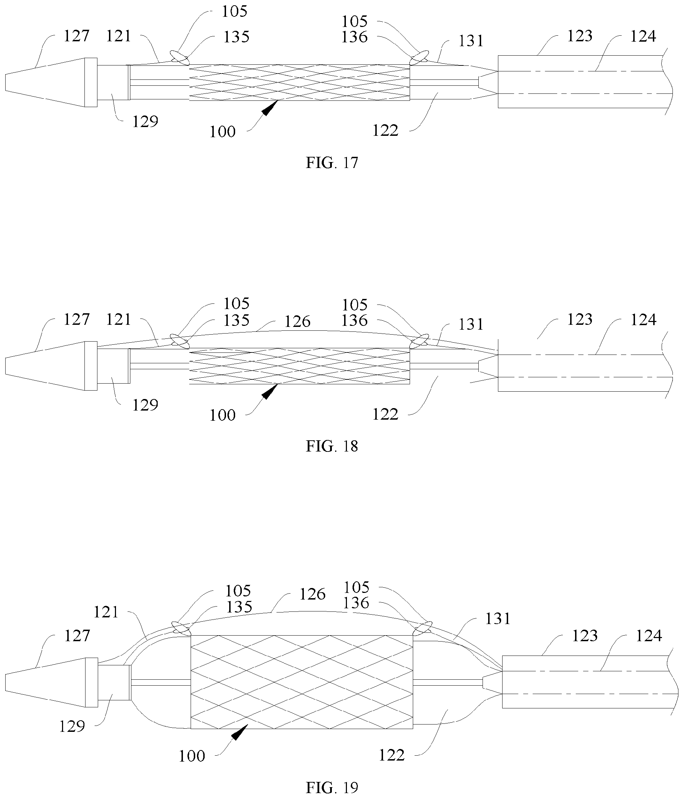

The adjustment string and the implantable medical device pass through each other, and the locking wire limit the release therebetween so as to achieve locking. Accordingly, referring to FIGS. 17 to 19, an embodiment of the present application discloses a delivery system for an implantable medical device, including a balloon catheter, and further includes:
a sheath 123 slidably arranged around the balloon catheter 120 for covering the implantable medical device 100;
an adjustment string for releasably securing the implantable medical device 100 over the balloon catheter 120, one end of the adjustment string can be fixed with the balloon catheter 120, and the other end has an eyelet and passed through the implantable medical device 100; and
a locking wire 126 having a relative locked state and an unlocked state, wherein in the locked state, the locking wire 126 passes through the implantable medical device 100 to prevent the implantable medical device 100 from detaching from the adjustment string, and in the unlocked state, the locking wire 126 is disengaged from the implantable medical device 100, allowing the eyelet to disengage from the implantable medical device 100, thereby releasing the implantable medical device 100.

The main difference between this embodiment and the above embodiments is that after the adjustment string and the implantable medical device pass through each other, the locking wire engages with the implantable medical device. For example, in one embodiment, the implantable medical device 100 is provided with a ring 105, and in the locked state, the ring 105 passes through the end of the corresponding adjustment string with an eyelet, and engages with the locking wire 126.

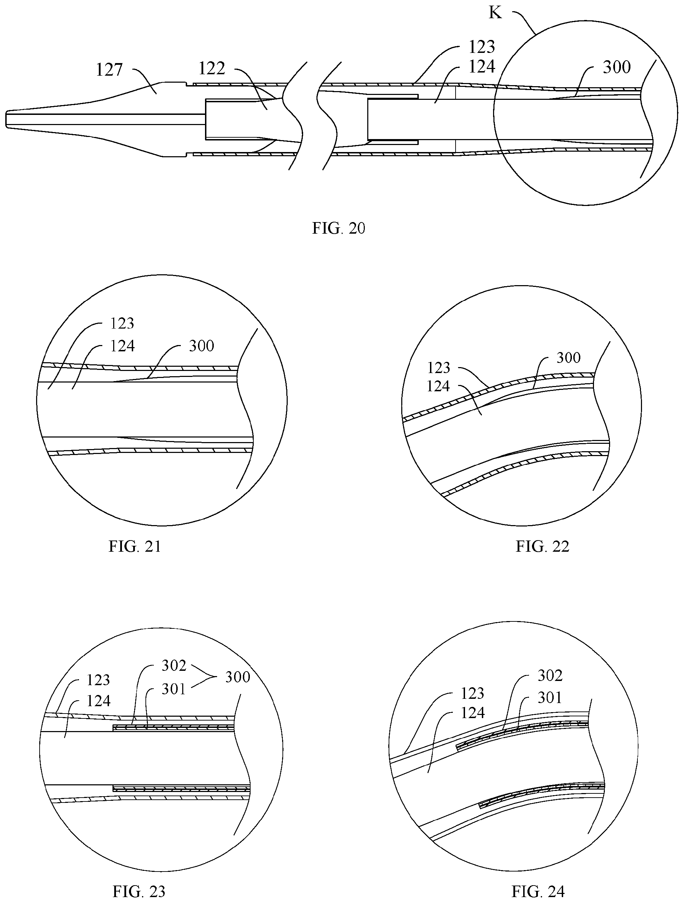

Referring to FIGS. 20 to 24, in one embodiment, the delivery system further includes a bending member 300 which is configured to control the distal portion of the balloon catheter 120 and change the orientation of the balloon catheter 120.

Regarding the relative position between the bending member 300 and the catheter body 124 in the radial direction, in some embodiments, the bending member 300 is located inside, outside, or sandwiched in the wall of the catheter body 124, and the distal portions of the bending member 300 and the catheter body 124 interact to make the balloon catheter 120 bend at the distal portion thereof.

The bending member 300 can be one of tube, wire or rod, or in combination, and the proximal end of the bending member 300 is connected to and controlled by the control handle 200. The distal portion of the bending member 300 can apply a force on the catheter body 124 to make the catheter body 124 bend.

The bending member 300 can act on the balloon catheter 120 directly or indirectly. In some of the following embodiments, the bending member 300 is configured to act on the catheter body 124, with various specific connecting methods of the bending member 300 with the catheter body 124. For example, the distal ends of the bending member 300 and the catheter body 124 can be fixedly connected or movably fitted.

Referring to FIG. 21 and FIG. 22, in one embodiment, the bending member 300 is a bending wire or a bending tube. The distal ends of the bending member 300 and the catheter body are fixed, while the proximal ends thereof are in a slide fit for bending the distal portion of the balloon catheter 120.

Since the distal portions of the bending member 300 and the catheter body 124 are fixed to each other, when the proximal end of one of the bending member 300 and the catheter body 124 slides relative to the other under force, the distal ends of the two will be bent, making the distal end of the entire delivery system bend, thereby changing the orientation of the balloon catheter 120 to adapt to the intervention path or lesion site. Taking the bending tube as an example, it can be arranged inside the catheter body or outside the catheter body.

Referring to FIG. 23 and FIG. 24, in one embodiment, the bending member 300 includes an inner bending tube 301 and an outer bending tube 302 surrounding the inner bending tube 301. The distal ends of the inner bending tube 301 and the outer bending tube 302 are fixed, and the proximal ends thereof are in a slide fit for bending the distal portion of the bending member 300 which, in turn, acts on the catheter body 124.

The bending member 300 uses inner and outer tubes, the relative movement of the proximal ends of which causes the distal ends thereof to bend, thereby driving the catheter body 124 to bend. The bending member 300 can be located inside or outside the catheter body 124. In the figures, the bending member 300 is located outside the catheter body 124. In addition, one of the two bending tubes can be replaced by a bending wire.

In this embodiment, at least one of the inner bending tube 301 and the outer bending tube 302 is movably connected to the control handle 200.

The control handle 200 mentioned above for realizing various distal controls can use the know technology to drive the corresponding components. The improved structure of the control handle 200 will be provided below, and the working principle will also be described in detail:

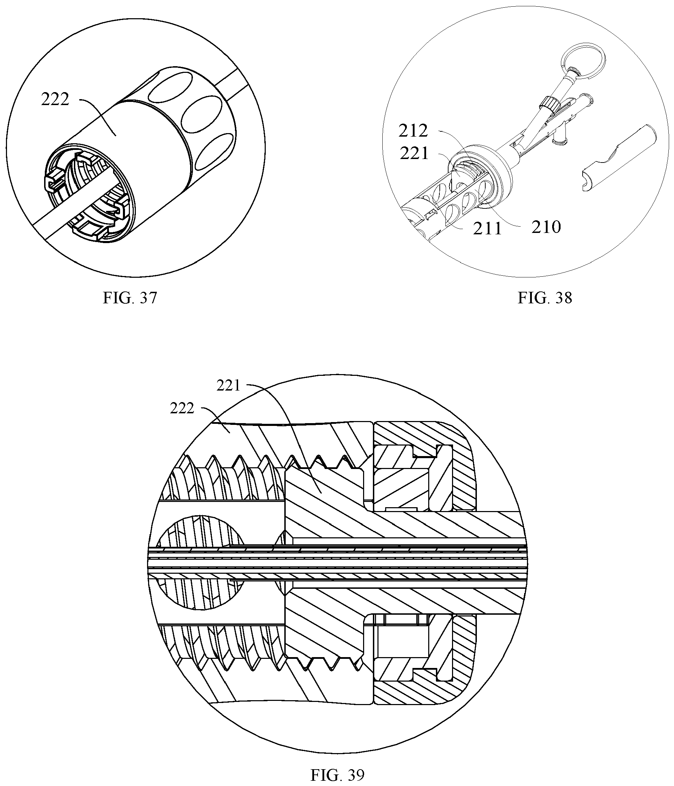

Referring to FIG. 16 and FIG. 27 to FIG. 42, the delivery system of the present application includes a control handle 200 for operating the implantable medical device. The control handle 200 includes a support 210 and a first driving assembly 220 mounted on the support 210. The first driving assembly includes:
a first mounting base 221 slidably arranged on the support 210; and
a first driving member 222 movably provided on the support 210, and in transmission fit with the first mounting base 221.

The first mounting base 221 includes a main body with a lumen, and ports provided on the main body and communicating with the lumen:
a distal port 261 for docking with the balloon catheter 120;
a driving port 262 for injecting fluid into the balloon catheter 120;
a limiting port 263 for threading the locking wire 126; and
a proximal port 264 for threading the guide wire.

The balloon catheter 120 extends through the support via tube(s) and is fixedly connected to the distal port 261. The movement of the first driving member 222 relative to the support drives the first mounting base 221 to slide, so as to drive the tube(s) connected with the balloon catheter 120 to slide, thereby adjusting the position of the balloon catheter 120 around the lesion site, optimizing the position for releasing the implantable medical device.

The first mounting base 221 is arranged at the proximal end of the control handle 200, which is a tube and has at least four tube openings corresponding to the ports.

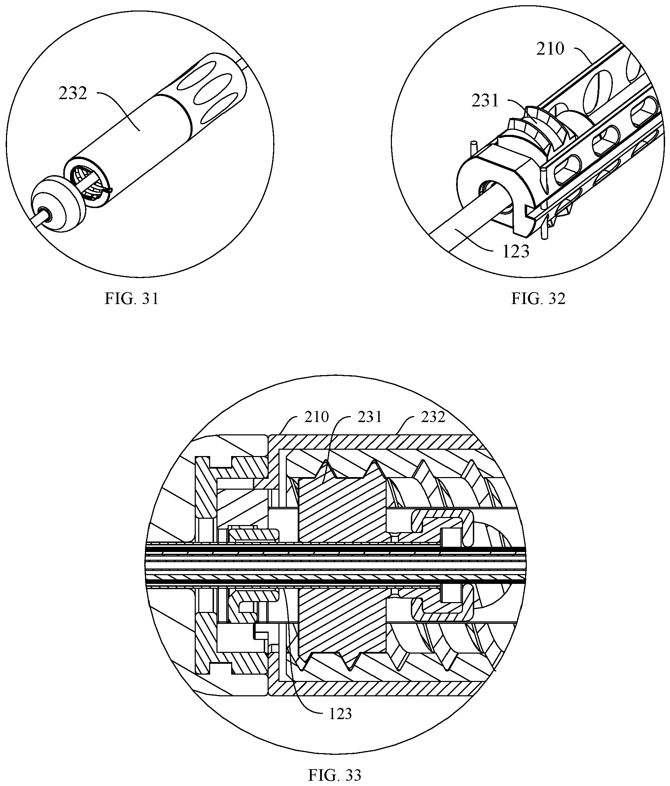

Referring to FIGS. 31 to 33, in one embodiment, the control handle 200 further includes a second driving assembly 230 installed on the support 210. The second driving assembly is located at the proximal side of the first driving assembly, and includes:
a second mounting base 231 slidably disposed on the support 210; and
a second driving member 232 movably installed on the support 210 and in transmission fit with the second mounting base 231.

The second mounting base 231 is used to connect the sheath 123, and the second driving member 232 moves relative to the support 210, driving the second mounting base 231 to drive the sheath 123 to slide so as to cover/expose the balloon catheter 120.

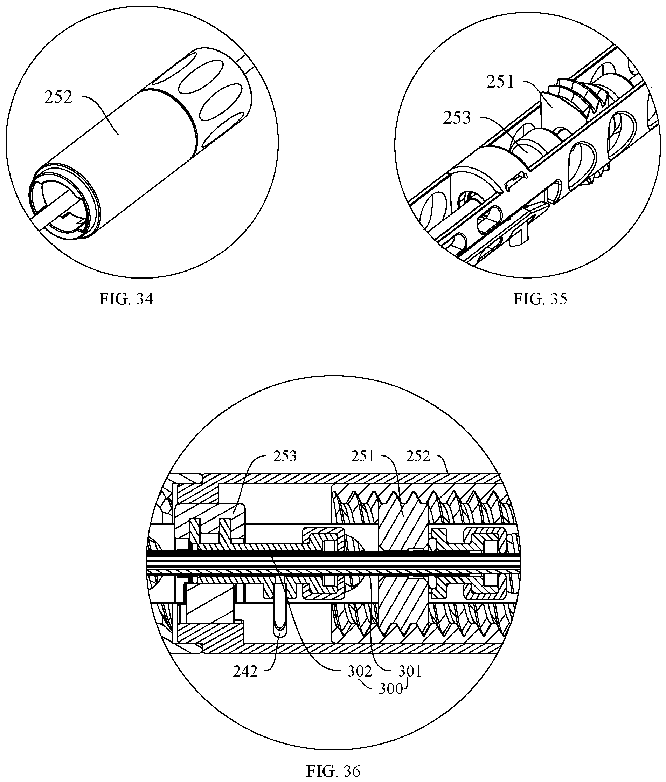

Referring to FIG. 34 to FIG. 36, in one embodiment, the control handle further includes a third driving assembly 250 installed on the support 210. The third driving assembly is located between the first and second driving assemblies, and includes:
a fixed mounting base 253;
a third mounting base 251 slidably disposed on the support 210; and
a third driving member 252 movably installed on the support 210 and in transmission fit with the third mounting base 251.

The outer bending tube 302 is connected to the fixed mounting base 253, and the inner bending tube 301 is connected to the third mounting base 251. When the third driving member 252 drives the third mounting base 251 to move relative to the fixed mounting base 253, the distal portion of the entire bending member bends, which in turn acts on the balloon catheter to create a bending effect. Regarding the transmission between the driving members and the corresponding mounting bases, as well as the fitting among the driving members, the mounting bases and the support, in one embodiment, the support 210 has an axial direction, and the support is provided with guide grooves 211 extending along the axial direction thereof, and the mounting bases are slidably provided in the corresponding guide grooves 211; and the driving members are respectively rotatably provided around the support, and in thread fit with the corresponding mounting bases.

In one embodiment, the support 210 has an axial direction. The first mounting base has a four-way structure with four side tubes, and the distal port, driving port, limiting port and proximal port are the corresponding tube openings of the respective side tubes. Along the axial direction of the support, the distal port and the proximal port are aligned with each other.

In order to reduce space occupation, the axes of the four side tubes are generally in the same plane. The side tubes corresponding to the driving port and the limiting port are located on two sides of the tube where the distal port is located, which is convenient for the tube docking.

In one embodiment, the end of the side tube where the driving port, the limiting port and the proximal port are located has an outer thread for convenience of connection with external pipeline.

In one embodiment, the proximal end of the catheter body 124 is in a sealing connection with the distal port, and the proximal end of the central tubing 133 extends out of the catheter body and then extends in the lumen of the first mounting base until in a sealing connection with the proximal port.

The driving port is communicated to the radial gap between the catheter body and the central tubing, and the fluid flows from the gap between the two to the distal balloon.

In one embodiment, after the proximal end of the locking wire 126 extends into the lumen of the first mounting base, it directly passes out of the first mounting base through the limiting port, or passes out of the first mounting base through the side wall of the side tube where the limiting port is located.

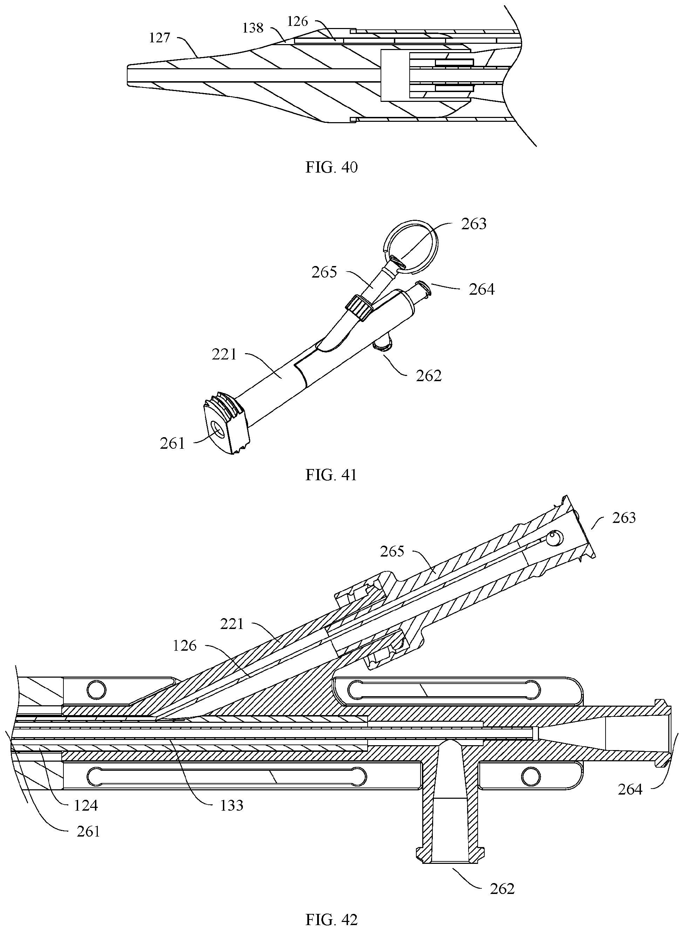

Referring to FIG. 42, the first mounting base is detachably connected with an operating member 265 at the limiting port 263, and the operating member 265 is fixedly connected to the locking wire 126. After the operating member 2 65 is detached from the first mounting base, it serves as an operating handle for the operator to hold to drive the locking wire 126 to move. When there is no need to drive the locking wire 126, the operating member 2 65 can be installed and fixed on the first mounting base. The operating member 265 can be fit with the first mounting base in a thread manner.

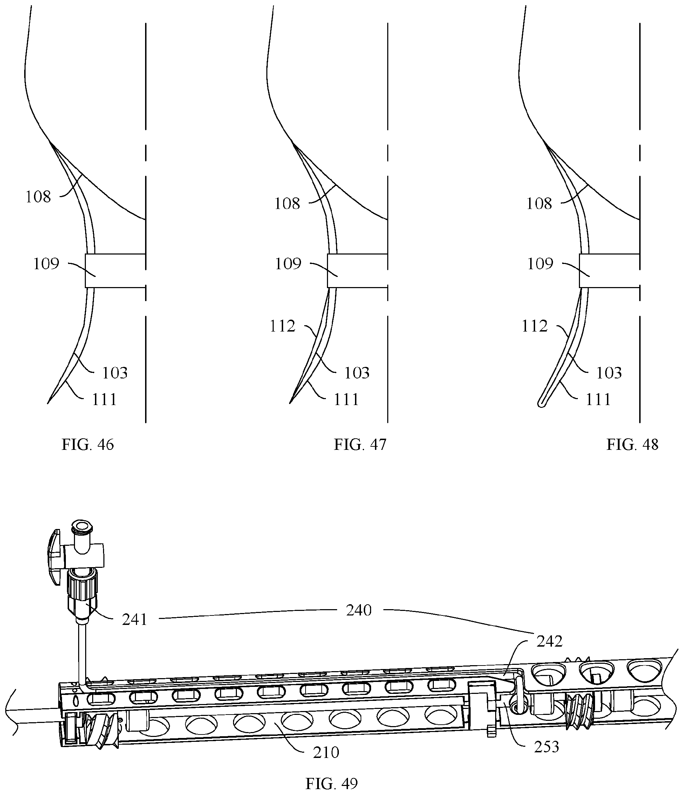

In one embodiment, the side tube where the limiting port is located extends obliquely toward the proximal end of the support, and the angle α between this side tube and the axis of the support satisfies 0°<α<60°. For example, 10 degrees<α<45 degrees, or 15 degrees<α<30 degrees. Referring to FIG. 49, in one embodiment, the control handle 200 includes an exhaust assembly 240, and the exhaust assembly includes:
a connector 241 and a liquid injection pipe 242, one end of the liquid injection pipe 242 is connected to the connector, and the other end is connected to the fixed mounting base 253.

For example, physiological saline can be used for exhaust. The physiological saline enters the fixed mounting base 253 through the exhaust assembly 240, and then enters the gaps between adjacent tubes through the openings provided in the walls of the tubes to discharge the air.

For convenience of the operation and reducing the interference on the operator, the injection pipe 242 extends from the fixed mounting base 253 within the control handle to the distal end of the control handle, and then passes out of the control handle and fits with the connector 241.

Now refer back to the above-mentioned configuration in which the distal ends of the bending member 300 and the catheter body 124 are movably fitted with each other.

Operate the proximal end of the bending member 300 by the control handle 200 to make the catheter body 124 bend the balloon catheter 120, and then stop operating the bending member to fix the proximal end of the bending member 300, keeping the distal end.

Move the balloon catheter 120 along the support 300 by the control handle 200 so that the distal end of the balloon catheter 120 moves along its own axis to adjust the position of the balloon catheter relative to the lesion site, thereby optimizing the release position of the implantable medical device.

The implantable medical device expands through the inflation of the balloon. However, if the implantable medical device has various radial dimensions in some locations, it would be difficult to use a conventional balloon to expand it. Even if a balloon with a customized shape is used, the implantable medical device will deform due to these location, resulting in unintended expanded effect.

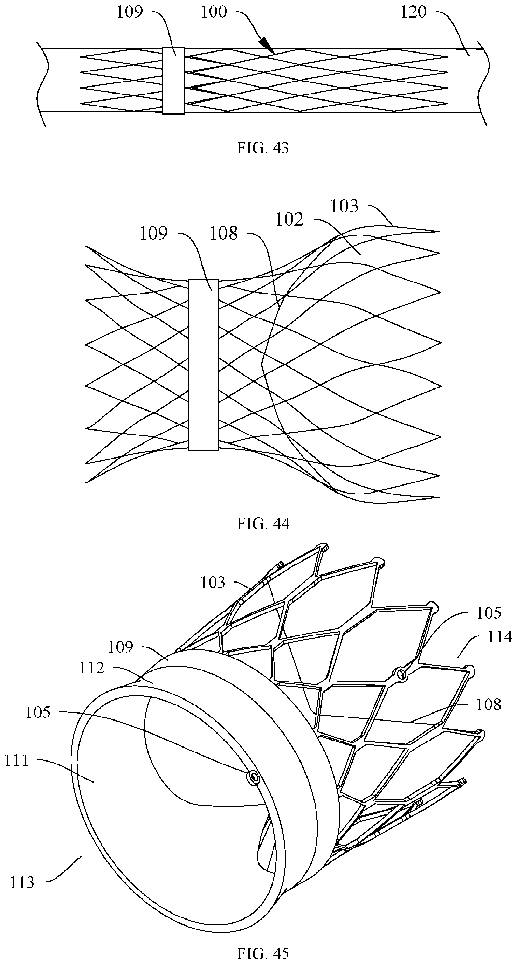

In order to solve the above problems, referring to FIG. 43 to FIG. 48, an embodiment of the present application provides an implantable medical device 100, including a stent 103 and leaflets 108 connected to the stent 103. The stent 103 has a radially deformable structure and has relative crimped and expanded states. The implantable medical device 100 further includes a restraint ring 109 that is connected to and around the stent 103 in the circumferential direction. The restraint ring 109 can restrict the radial deformation of the stent 103 when the stent 103 transforms into the expanded state.

The interior of the stent 103 is configured as a blood flow channel extending axially, and 2, 3 or 4 leaflets 108 are provided, which cooperate with each other to open or close the blood flow channel.

In the circumferential direction of the stent, the restraint ring 109 is installed and positioned around the stent 103 in one circle, so that the stent 103 assumes a waist structure having a relatively reduced diameter in the expanded state. In the radial direction of the stent, the restraint ring 109 is disposed on the outer periphery or the inner wall of the stent or undulating through the inner and outer sides of the stent. In the axial direction of the stent 103, the restraint ring 109 is located in the middle of the stent or adjacent to the inflow side of the stent or adjacent to the inflow side of the valve leaflets. The restraint ring 109 can be provided around the stent in various methods. For example, the restraint ring 109 can be sewn on the struts for the hollow structures of the stent.

Depending on the position of the restraint ring 109 in the axial direction of the stent 103, the position of the diameter reduced portion varies. In the axial direction of the stent, the stent 103 includes a restrained section corresponding to the restraint ring 109, and free sections on two sides of the restrained section. In the expanded state, the restrained section has a smaller outside diameter than the free sections. The restraint ring 109 changes the expansion deformation of the restrained section relative to the free sections, and the deformation of the restrained section is smaller than that of the free sections per unit time. Similarly, the connecting sections between the restrained section and the free sections also deform slowly under the restraint of the restraint ring 109, and eventually bend and transition between the restrained section and the free sections.

In this embodiment, the restraint ring 109 has a loop structure. The loop structure can be formed by fixing two ends of an elongated strip to each other, or in one piece, among others.

In some embodiments, in the axial direction of the stent, the width of the restraint ring 109 ranges within 2 mm to 30 mm (the width of the restraint ring in a flattened state), for example, 5 mm to 20 mm, or for example 5 mm to 15 mm, or for another example 8 mm. The restraint ring 109 is a flexible material that can be compressed along with the stent 103.

The restraint ring 109 has a low stretch rate, such as being made of PTFE and the like, so that during the expansion of the stent 103, the restraint ring 109 can first expand along with the stent 103, and then the expansion can be slowed down or stopped when the restraint ring 109 reaches its elastic limit.

The restraint ring 109 can alternatively be made of metal wire, which can be formed by fixing two ends of the metal wire to each other, or in one piece, and the cross-sectional shape of the metal wire can be circular, elliptical, etc.

In one embodiment, the stent 103 is a mesh cylinder with a hollow structure and has an axial direction. Depending on the blood flow direction, one end of the stent in the axial direction is the inflow side 113 and the other end is the outflow side 114. The implantable medical device 100 further includes a first covering film 111 connected to the inner side of the stent, which is connected to the inflow side of the valve leaflets 108.

The first covering film 111 is circumferentially arranged within the stent 103 in a circle, and is connected to the connection between the leaflets 108 and the stent 103 for closing the hollow structures. In one embodiment, the implantable medical device further includes a second covering film 112 connected to the outside of the stent, and the second covering film 112 is connected to the inflow side of the restraint ring 109.

The second covering film 112 covers the outer periphery of the stent 103, and cooperates with the first covering film 111 to prevent peripheral leakage. Like the first covering film 111, the second covering film 112 can be fixed to the stent 103 by sewing.

The first covering film 111 and the second covering film 112 extend to the inflow side of the stent 103, and connected to each other to cover the inflow side edge of the stent 103.

For convenience of the assembly, in one embodiment, the first covering film 111 and the second covering film 112 are formed in one piece.

In other words, the two covering films are provided by one single film, which is bent and folded in half at the inflow side edge of the stent 103 to form the first covering film 111 and the second covering film 112, which are fixedly connected to the stent 103 respectively.

Preferably, the fold of the covering film is also fixedly connected to the stent 103.

Referring back to the foregoing, the covering film is provided with holes for the rings 105 on the stent 103 to expose.

Further preferably, one of the first covering film 111 and the second covering film 112 extends to the inflow side of the stent 103 and then be folded over the inflow side edge of the stent 103, and further extends to connect with the outflow side of the other film.

In the crimped state of the stent 103, the covering films will overlap with each other at the inflow side, which increases the radial thickness and interferes with the movement of the sheath 123, affecting the operation of the sheath 123.

In order to solve the above problem, the first covering film 111 and /or the second covering film 112 has cut areas adjacent to the inflow side edge of the stent, and the position of the cut areas match the inflow side edge of the stent 103 in shape.

There is a gap between two adjacent cut areas, and the gap gradually decreases during the crimping process of the stent 103, which reduces the overlaps of the covering films at the inflow side and thus facilitates the movement of the sheath 123.

In one embodiment, the inflow side of the stent 103 includes a plurality of cells 102 distributed in the circumferential direction, and gaps are defined between adjacent cells 102 toward the inflow side. The cut areas match the gaps in position.

In one embodiment, the edges of the first covering film 111 and / or the second covering film 112 adjacent to the inflow side of the stent have a zigzag structure with teeth, and each cut area is located between adjacent teeth. The two sides of the cut area are stitched to the corresponding portions of the stent.

An embodiment of the present application provides an implantable system, including an implantable medical device and a delivery system cooperating with the implantable medical device.

The implantable medical device and the delivery system can use or combine any of the above embodiments. The implantable medical device is radially crimped against the outer peripheral wall of the balloon catheter in the crimped state. In the axial direction of the balloon catheter, the balloon catheter is releasably fixed with adjustment strings with eyelets at the two ends of the implantable medical device, with the ends of the adjustment strings with the eyelets threading through the two axial ends of the implantable medical device. The delivery system further includes a locking wire that is slidably arranged along the balloon catheter, which passes through the eyelets to prevent the adjustment strings from being detached from the implantable medical device.

Certain distance can be remained between the eyelets of the two adjustment strings and the ends of the implantable medical device. This distance can be adjusted manually, so that the relative position of the implantable medical device in the axial direction of the balloon catheter can be controlled. The distance can be zero, thereby restricting the movement of implantable medical device over the balloon catheter.

An embodiment of the present application further provides a release method for an implantable medical device. The implantable medical device 100 is pre-fixed on the delivery system. The release method includes:
driving the sheath 123 to move toward the proximal side of the delivery system to expose the implantable medical device 100;
pulling the locking wire 126 out from the eyelets of the adjustment strings;
inflating the balloon 122 to drive the implantable medical device 100 to deform to the expanded state, and withdrawing the adjustment strings out from the implantable medical device 100; and
deflating the balloon 122, and moving the balloon catheter 120 toward the proximal side of the delivery system to separate from the implantable medical device 100.

The delivery system can use or combine any of the above embodiments.

After the implantable medical device 100 is delivered to the lesion, the proximal end of the delivery system can control the distal sheath 123 through the control handle to slide proximally along the axial direction of the balloon catheter, to gradually expose the implantable medical device 100 until the sheath 123 is completely withdrawn from the radial expansion path of the implantable medical device 100.

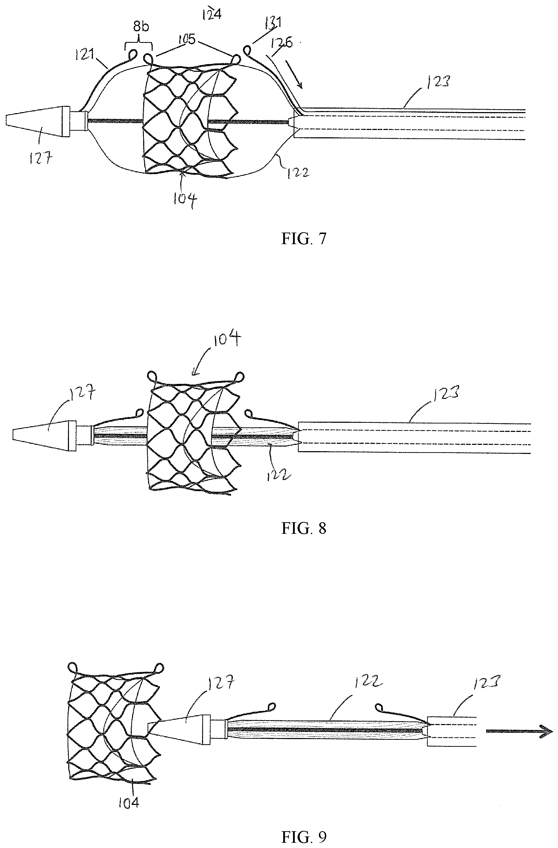

The locking wire 126 is withdrawn approximately in the axial direction of the balloon catheter, and the first eyelet 135 and the second eyelet 136 will automatically and completely disengage from their corresponding rings 105. The locking area 8b illustrates the disengagement between the eyelets and their rings 105. Next, in FIG. 8, the locking wire 126 is completely withdrawn and the balloon 122 is deflated and turns into the deflated state. Finally, in FIG. 9, the balloon catheter 120 is withdrawn.

If the locking wire 126 is sufficiently flexible so as not to impede the expansion of the implantable medical device 100, the locking wire 126 can be withdrawn after the implantable medical device 100 is expanded, so that during the expansion, the axial position of the device relative to the balloon catheter can be fixed.

After the expansion, the fluid is discharged, the balloon 122 is deflated and detached from the implantable medical device 100, and finally the delivery system is withdrawn from the human body.

The order of the steps in the above release method is not strictly limited. For example, the following method can be alternatively used:
driving the sheath 123 to move toward the proximal side of the delivery system to expose the implantable medical device 100;
inflating the balloon 122 to drive the implantable medical device 100 to deform to the expanded state, and withdrawing the adjustment strings out from the implantable medical device 100;
deflating the balloon 122, and pulling the locking wire 126 out from the eyelets of the adjustment strings; and
moving the balloon catheter 120 toward the proximal side of the delivery system to separate from the implantable medical device 100.

This method differs from the aforementioned release method in: during the expansion of the stent, the locking wire 126 deforms and bends adaptively (during the deformation, the distal end or the proximal end of the locking wire can moved to compensate for the length change of the locking wire corresponding to the periphery of the stent), but it has never disengaged from the eyelets to limit the movement of the stent in the axial direction. After the expansion is completed and the balloon 122 is deflated, the locking wire 126 is driven out of the eyelets, and then the balloon catheter is detached from the implantable medical device by controlling the delivery system, and withdrawn from the human body.

An embodiment of the present application further provides a loading method for an implantable medical device, for securing the implantable medical device to a delivery system, wherein the delivery system can use or combine any of the above embodiments.

The loading method includes:
placing the implantable medical device 100 in the expanded state over the balloon 122 in the deflated state;
radially crimping the implantable medical device 100 into a crimped state;
threading the end of the adjustment string with the eyelet through the implantable medical device 100;
threading the locking wire through the eyelet; and
sliding the sheath 123 toward the distal side of the delivery system until it covers the implantable medical device 100.

The implantable medical device 100 in the expanded state can be uniformly crimped in the circumferential direction by using existing devices, such as a crimping device, until it transforms into the crimped state.

Referring to FIG. 3A, the first and second eyelets 135 and 136 of the first and second strings 121 and 131 are threaded through the rings 105 at the distal end 106 and the proximal end 107, respectively, of the heart valve assembly 104.

This can be done manually by a person during the assembly process or using specialized tools. Once this is completed, the locking wire 126 is advanced through a lumen 137 that is defined between the sheath 123 and the catheter body 124, and exits the distal end 138 of the sheath 123.

Next, as shown in FIG. 3B, the locking wire 126 is advanced through the first and second eyelets 135 and 136 and into the receiving hole 138 inside the guide head 127. The locking location LK in FIG. 3B illustrates a locking knot where the locking wire 126 passes through the eyelets after the eyelets have been threaded through the rings 105. The hole inside the guide head 127 should have a certain depth so that the locking wire 126 can be securely locked inside the guide head 127.

An embodiment of the present application further provides a method for securing an implantable medical device over a balloon catheter, including the following steps:
providing an implantable medical device having a tubular device body that has a first end and a second end, with a first ring provided at the first end and a second ring provided at the second end;
providing a balloon catheter having:
   a catheter body that has a guide head,
   a balloon provided adjacent to, and proximal to, the guide head, and having a distal end and a proximal end,
   a first string having a first eyelet and secured to the balloon catheter at a position adjacent the distal end of the balloon catheter, and
   a second string having a second eyelet and secured to the balloon catheter at a position adjacent the proximal end of the balloon catheter;
providing a sheath for sliding movement over the catheter body and the balloon, the sheath having a distal end;
positioning the implantable medical device in its expanded state over the deflated balloon;
radially crimping the implantable medical device over the deflated balloon;
threading the first and second eyelets of the first and second strings, respectively, through the first and second rings, respectively;
advancing a locking wire through the first and second eyelets and into the guide head; and
advancing the sheath over the crimped medical device to the guide head to completely cover the crimped medical device.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification. When the technical features in different embodiments are shown in the same drawing, it can be considered that the drawing also discloses a combined embodiment of various embodiments involved.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure.

## Claims

1. A delivery system for implantable medical device, comprising a balloon catheter, and further comprising:
a sheath in sliding fit over the balloon catheter and for covering the implantable medical device;
an adjustment string for releasably securing the implantable medical device over the balloon catheter, one end of the adjustment string is capable of being fixed to the balloon catheter, and the other end is capable of passing through the implantable medical device and has an eyelet; and
a locking wire having relative locked and unlocked states, wherein in the locked state, the locking wire passes through the eyelet to restrain the implantable medical device, and in the unlocked state, the locking wire disengages from the eyelet to release the implantable medical device.

2. The delivery system for implantable medical device according to claim 1, wherein the delivery system further comprises a control handle, to which all of the balloon catheter, the sheath and the locking wire extend, and the balloon catheter, the sheath, and the locking wire are movable relative to each other.

3. The delivery system for implantable medical device according to claim 2, wherein the balloon catheter comprises:
a guide head provided with a receiving hole, into which an end of the locking wire extends in the locked state;
a balloon adjacent to, and proximal to, the guide head, an outer periphery of the balloon being configured as a loading area for the implantable medical device; and
a catheter body communicated with the balloon and extending toward a proximal end of the delivery system.

4. The delivery system for implantable medical device according to claim 3, wherein the balloon catheter further comprises:
a central tubing passing through the catheter body, wherein one end of the central tubing extends toward the proximal end of the delivery system, and the other end passes through the balloon and is connected with the guide head.

5. The delivery system for implantable medical device according to claim 3, wherein the locking wire is configured to extend from a lumen defined between the sheath and the catheter body toward the proximal end of the delivery system.

6. The delivery system for implantable medical device according to claim 3, wherein the locking wire is configured to extend from a lumen defined in the catheter body toward the proximal end of the delivery system.

7. The delivery system for implantable medical device according to claim 6, wherein a tube wall of the catheter body has a through hole, and a distal end of the locking wire is configured to extend outside the catheter body via the through hole to engage with the adjustment string.

8. The delivery system for implantable medical device according to claim 7, wherein the tube wall of the catheter body has a radially concave area, and the through hole is opened in a proximal portion of the radially concave area.

9. The delivery system for implantable medical device according to claim 1, wherein there is only one joint between the adjustment string and the locking wire.

10. The delivery system for implantable medical device according to claim 1, wherein there are at least two joints between the adjustment string and the locking wire, and wherein at least one of the joints restricts movement of the implantable medical device in a distal direction, and at least another one restricts movement of the implantable medical device in a proximal direction.

11. The delivery system for implantable medical device according to claim 4, wherein one or more adjustment strings are provided, and each adjustment string is provided with one or more eyelets.

12. The delivery system for implantable medical device according to claim 11, wherein the eyelets are arranged in pairs, and in each pair, one of the eyelets is close to a distal end of the balloon catheter, and the other is close to a proximal end of the balloon catheter.

13. The delivery system for implantable medical device according to claim 11, wherein the adjustment string comprises:
a first adjustment string having a first eyelet and connected to the balloon catheter and adjacent to a distal end of the balloon catheter; and
a second adjustment string having a second eyelet and connected to the balloon catheter and adjacent to a proximal end of the balloon catheter;
wherein in the locked state, the locking wire passes through the first eyelet and the second eyelet simultaneously.

14. The delivery system for implantable medical device according to claim 13, wherein the first adjustment string and the second adjustment string are respectively fixedly connected to the balloon catheter.

15. The delivery system for implantable medical device according to claim 13, wherein the first adjustment string is fixed to the guide head.

16. The delivery system for implantable medical device according to claim 13, wherein the second adjustment string is fixedly provided on an outer periphery the catheter body.

17. The delivery system for implantable medical device according to claim 16, wherein the outer periphery of the catheter body is provided with a limiting step, and the second adjustment string is limited by the limiting step.

18. The delivery system for implantable medical device according to claim 13, wherein the first adjustment string and the second adjustment string are both movably connected to the balloon catheter.

19. The delivery system for implantable medical device according to claim 13, wherein the first adjustment string and the second adjustment string are connected by a connecting string.

20. The delivery system for implantable medical device according to claim 13, wherein at least one of the first adjustment string and the second adjustment string is formed with the connecting string in one piece.

21. The delivery system for implantable medical device according to claim 20, wherein the connecting string extends toward the proximal end of the delivery system.

22. The delivery system for implantable medical device according to claim 21, wherein the connecting string extends toward the proximal end of the delivery system through the central tubing and can be pulled relative to the balloon catheter.

23. The delivery system for implantable medical device according to claim 13, wherein one of the first adjustment string and the second adjustment string is configured as a fixed adjustment string fixedly connected to the balloon catheter, and the other is configured as a movable adjustment string that can be pulled relative to the balloon catheter, and wherein a middle sheath is movably threaded through a lumen defined between the sheath and the catheter body, and the movable adjustment string is connected to the middle sheath.

24. The delivery system for implantable medical device according to claim 23, wherein the central tubing has a plurality of lumens, through one of which the movable adjustment string extends.

25. The delivery system for implantable medical device according to claim 24, wherein one of the plurality of lumens is a central lumen, and at least another one is an eccentrically defined second lumen, through which the movable adjustment string extends.

26. The delivery system for implantable medical device according to claim 25, wherein two openings are defined in a lumen wall of the second lumen, and the first adjustment string and the second adjustment string extend out of the central tubing through the corresponding openings.

27. The delivery system for implantable medical device according to claim 11, wherein the implantable medical device is provided with a ring, and in the locked state, an end of the adjustment string having the eyelet engages with the locking wire after passing through the corresponding ring.

28. The delivery system for implantable medical device according to claim 27, wherein at least two rings are provided, which are provided at two opposite ends of the implantable medical device, or at least one of the two rings is provided adjacent to the opposite end of the implantable medical device.

29. The delivery system for implantable medical device according to claim 27, wherein the ring is provided using at least one of the following methods:
a, configured as in independent member and fixed to the implantable medical device;
b, opening a hole in the implantable medical device; and
c, directly using space of the implantable medical device itself.

30. The delivery system for implantable medical device according to claim 27, wherein the implantable medical device has a tubular device body, and the end of the adjustment string having the eyelet is threaded from an interior of the device body radially towards an exterior of the device body.

31. The delivery system for implantable medical device according to claim 27, wherein the locking wire is a metal rod.

32. The delivery system for implantable medical device according to claim 4, wherein the delivery system further comprises a bending member which is located inside, outside, or sandwiched in a wall of the catheter body, and distal portions of the bending member and the catheter body are configured to interact with each other so as to bend a distal portion of the balloon catheter.

33. The delivery system for implantable medical device according to claim 32, wherein a proximal end of the bending member is connected to and controlled by the control handle.

34. The delivery system for implantable medical device according to claim 32, wherein the bending member is a bending wire or a bending tube, and wherein distal ends of the bending member and the catheter body are fixed with each, and proximal ends are in sliding fit so as to bend the distal portion of the balloon catheter.

35. The delivery system for implantable medical device according to claim 4, wherein the control handle for operating the implantable medical device comprises a support and a first driving assembly provided on the support, and wherein the first driving assembly comprises:
a first mounting base slidably arranged on the support; and
a first driving member movably arranged on the support and in transmission fit with the first mounting base; and
wherein the first mounting base comprises a main body with a lumen, and ports opened in the main body and communicated with the lumen:
a distal port for docking with the balloon catheter;
a driving port for injecting fluid into the balloon catheter;
a limiting port for threading the locking wire; and
a proximal port for threading the guide wire.

36. The delivery system for implantable medical device according to claim 35, wherein the control handle further comprises a second driving assembly provided on the support, the second driving assembly is proximal to the first driving assembly, and the second driving assembly comprises:
a second mounting base slidably arranged on the support; and
a second driving member movably arranged on the support and in transmission fit with the second mounting base.

37. The delivery system for implantable medical device according to claim 36, wherein the control handle further comprises a third driving assembly provided on the support, the third driving assembly is between the first and second driving assemblies, and the third driving assembly comprises:
a fixed mounting base;
a third mounting base slidably arranged on the support; and
a third driving member movably arranged on the support, and in transmission fit with the third mounting base.

38. The delivery system for implantable medical device according to claim 37, wherein the support has an axial direction, the support is provided with guide grooves extending along the axial direction, and the mounting bases are slidably arranged in the corresponding guide grooves; and the driving members are respectively rotatably provided around the support, and in thread fit with the corresponding mounting bases.

39. The delivery system for implantable medical device according to claim 38, wherein the support has an axial direction, the first mounting base is a four-way structure with four side tubes, and the distal port, the driving port, the limiting port and the proximal port are the corresponding tube openings of the respective side tubes; and
the distal port and the proximal port are aligned with each other in the axial direction of the support.

40. The delivery system for implantable medical device according to claim 39, wherein axes of the four side tubes are substantially in the same plane.

41. The delivery system for implantable medical device according to claim 40, wherein a proximal end of the catheter body is in sealing connection with the distal port, and a proximal end of the central tubing extends out of the catheter body and through the lumen of the first mounting base until in sealing connection with the proximal port; and
the driving port communicates to a radial gap between the catheter body and the central tubing.

42. The delivery system for implantable medical device according to claim 41, wherein a proximal end of the locking wire is configured to extend into the lumen of the first mounting base, and then directly out of the first mounting base through the limiting port, or out of the first mounting base through a side wall of the side tube where the limiting port is located.

43. The delivery system for implantable medical device according to claim 42, wherein the side tube where the limiting port is located extends obliquely toward a proximal end of the support with an angle α relative to an axis of the support satisfies 0 degree<α< 60 degrees.

44. A delivery system for implantable medical device, comprising a balloon catheter, further comprising:
a sheath in sliding fit over the balloon catheter and for covering the implantable medical device;
an adjustment string for releasably securing the implantable medical device over the balloon catheter, one end of the adjustment string is capable of being fixed to the balloon catheter, and the other end has an eyelet and is capable of passing through the implantable medical device; and
a locking wire having relative locked and unlocked states, wherein in the locked state, the locking wire passes through the implantable medical device to limit detachment of the implantable medical device from the adjustment string, and in the unlocked state, the locking wire is detached from the implantable medical device, allowing the eyelet to disengage from and release the implantable medical device.

45. The delivery system for implantable medical device according to claim 44, wherein the implantable medical device is provided with a ring, and in the locked state, the ring passes through an end of the corresponding adjustment string having the eyelet and engages with the locking wire.

46. An implantable medical device, comprising a stent and leaflets connected to the stent, wherein the stent is a radially deformable structure and has relative crimped and expanded states, and the implantable medical device further comprises a restraint ring connected to and around the stent, and the restraint ring is configured to restrain a radial deformation of the stent when the stent transforms into the expanded state.

47. The implantable medical device according to claim 46, wherein the restraint ring is arranged outside, inside, or undulating through the inside and outside of the stent.

48. The implantable medical device according to claim 46, wherein in an axial direction of the stent, the restraint ring is located at a middle of the stent or adjacent to an inflow side of the stent.

49. The implantable medical device according to claim 48, wherein in the axial direction of the stent, the restraint ring is located adjacent to an inflow side of the leaflets.

50. The implantable medical device according to claim 46, wherein in an axial direction of the stent, the stent comprises a restrained section corresponding to the restraint ring and free sections, respectively, on two sides of the restrained section, and in the expanded state, the restrained section has a smaller outer diameter than the respective free sections.

51. The implantable medical device according to claim 46, wherein the stent is a mesh cylinder with a hollow structure and has an axial direction, depending on blood flow direction, one end of the stent in the axial direction is configured as an inflow side, the other end is configured as an outflow side, and an interior of the stent is configured as a blood flow channel extending in the axial direction, and 2, 3 or 4 leaflets are provided and cooperate with each other to open or close the blood flow channel.

52. The implantable medical device according to claim 51, wherein the implantable medical device further comprises a first covering film connected to an inside of the stent, and the first covering film is connected to the inflow side of the leaflets.

53. The implantable medical device according to claim 52, wherein the implantable medical device further comprises a second covering film connected to an outside of the stent, and the second covering film is connected on an inflow side of the restraint ring.

54. The delivery system for implantable medical device according to claim 53, wherein the first covering film and the second covering film are formed in one piece.

55. The implantable medical device according to claim 53, wherein both the first covering film and the second covering film extend to the inflow side of the stent, and are connected with each other to cover an inflow side edge of the stent.

56. The implantable medical device according to claim 53, wherein one of the first covering film and the second covering film extends to the inflow side of the stent and folds back to cover an inflow side edge of the stent, and further extends to connect with an outflow side of the other covering film.

57. The implantable medical device according to claim 56, wherein the first covering film and/or the second covering film have cut areas at side edge thereof adjacent to the inflow side of the stent, and the cut areas are matched with the inflow side edge of the stent in shape.

58. The implantable medical device according to claim 57, wherein the inflow side of the stent comprises a plurality of cells arranged in a circumferential direction, with gaps located between adjacent cells and facing the inflow side, and the cut areas are matched with the corresponding gaps.

59. The implantable medical device according to claim 58, wherein the first covering film and/or the second covering film have a zigzag structure with teeth at the side edge thereof adjacent to the inflow side of the stent, and the cut area is located between adjacent teeth and two sides thereof are sewed and connected with the corresponding portions of the stent.

60. An implantable system, comprising an implantable medical device and a delivery system engaged with the implantable medical device; wherein the delivery system is the delivery system for implantable medical device according to any one of claims 1 to 45.

61. A releasing method for implantable medical device which is preset in the delivery system according to any one of claims 1 to 45, comprising:
driving the sheath toward a proximal end of the delivery system to expose the implantable medical device;
pulling the locking wire out from the eyelet of the adjustment string;
inflating the balloon to drive the implantable medical device to deform to an expanded state, and withdrawing the adjustment string out from the implantable medical device; and
deflating the balloon, and moving the balloon catheter toward the proximal end of the delivery system to separate from the implantable medical device.

62. A loading method for implantable medical device for securing the implantable medical device to the delivery system according to any one of claims 1 to 45, comprising:
placing the implantable medical device in an expanded state over a deflated balloon;
radially crimping the implantable medical device into a crimped state;
threading an end of the adjustment string with the eyelet through the implantable medical device;
threading the locking wire through the eyelet; and
sliding the sheath toward a distal end of the delivery system until it covers the implantable medical device.

63. A method for securing an implantable medical device over a balloon catheter, comprising the steps of:
providing the implantable medical device having a tubular device body that has a first end and a second end, with a first ring provided at the first end and a second ring provided at the second end;
providing a balloon catheter having:
a catheter body that has a guide head,
a balloon provided adjacent to, and proximal to, the guide head, and having a distal end and a proximal end,
a first string having a first eyelet and secured to the balloon catheter at a position adjacent a distal end of the balloon catheter, and
a second string having a second eyelet and secured to the balloon catheter at a position adjacent a proximal end of the balloon catheter;
providing a sheath for sliding movement over the catheter body and the balloon, the sheath having a distal end;
positioning the implantable medical device in its expanded state over the deflated balloon;
radially crimping the implantable medical device over the deflated balloon;
threading the first and second eyelets of the first and second strings, respectively, through the first and second rings, respectively;
advancing a locking wire through the first and second eyelets and into the guide head; and
advancing the sheath over the crimped implantable medical device to the guide head to completely cover the crimped medical device;
wherein the step of advancing a locking wire includes the step of advancing the locking wire through a lumen defined between the sheath and the catheter body to exit the distal end of the sheath.

64. The method for securing an implantable medical device over a balloon catheter according to claim 63, wherein the step of threading the first and second eyelets includes the step of locking each of the first and second eyelets at the first and second rings, respectively.

65. The method for securing an implantable medical device over a balloon catheter according to claim 63, wherein each of the first and second rings is provided as an independent ring and attached to the first and second ends, respectively, of the implantable medical device.

66. The method for securing an implantable medical device over a balloon catheter according to claim 63, wherein each of the first and second rings is provided as a rounded tip at the first and second ends, respectively, of the implantable medical device.

67. The method for securing an implantable medical device over a balloon catheter according to claim 63, wherein each of the first and second rings is provided as an opening in an apex at the first and second ends, respectively, of the implantable medical device.

68. The method for securing an implantable medical device over a balloon catheter according to claim 63, further including providing a connecting wire that connects the first and second strings.

69. The method for securing an implantable medical device over a balloon catheter according to claim 63, further including providing a central lumen extending through the balloon, and wherein the connecting wire extends through the central lumen.

70. The method for securing an implantable medical device over a balloon catheter according to claim 69, further including providing a middle sheath that extends through a lumen defined between the sheath and the catheter body, with the second string attached to a distal end of the middle sheath.

71. The method for securing an implantable medical device over a balloon catheter according to claim 63, wherein a method of implanting the implantable medical device at a location in a human anatomy, and wherein the implantable medical device has been secured to the deflated balloon of the balloon catheter, comprising the steps of:
withdrawing the sheath in a proximal direction to expose the implantable medical device;
withdrawing the locking wire in a proximal direction;
expanding the balloon, with the expanding balloon causing the first and second strings to be withdrawn through the first and second rings, respectively;
deflating the balloon; and
withdrawing the balloon catheter in a proximal direction.
